(19) Europäisches Patentamt
European Patent Office
Office européen des brevets

(11) EP 1 078 928 B1

(12) # FASCICULE DE BREVET EUROPEEN

(45) Date de publication et mention de la délivrance du brevet: **12.05.2004 Bulletin 2004/20**

(21) Numéro de dépôt: **00402359.4**

(22) Date de dépôt: **25.08.2000**

(51) Int Cl.[7]: **C07D 491/048**, A61K 31/4355, A61K 31/4365, A61K 31/437, C07D 495/04, C07D 471/04, A61P 25/00 // (C07D491/048, 307:00, 221:00), (C07D495/04, 333:00, 221:00), (C07D471/04, 221:00, 209:00)

(54) **Dérivés de la pyridine, leur procédé de préparation et les compositions pharmaceutiques qui les contiennent**

Pyridinderivate, Verfahren zu ihrer Herstellung und diese enthaltende pharmazeutische Zubereitungen

Pyridine derivatives, process for their preparation and pharmaceutical compositions containing them

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **27.08.1999 FR 9910834**

(43) Date de publication de la demande: **28.02.2001 Bulletin 2001/09**

(73) Titulaire: **LES LABORATOIRES SERVIER
92200 Neuilly sur Seine (FR)**

(72) Inventeurs:
- **Peglion, Jean-Louis
78110 le Vesinet (FR)**
- **Dessinges, Aimée
92500 Rueil Malmaison (FR)**
- **Poitevin, Christophe
75019 Paris (FR)**
- **Millan, Mark
78230 le Pecq (FR)**
- **Dekeyne, Anne
78470 Saint Remy les Chevreuses (FR)**

(56) Documents cités:
**FR-A- 2 738 823**
**FR-A- 2 761 068**
**FR-A- 2 761 071**

Remarques:
Le dossier contient des informations techniques présentées postérieurement au dépôt de la demande et ne figurant pas dans le présent fascicule.

EP 1 078 928 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux dérivés de la pyridine, leur procédé de préparation et les compositions pharmaceutiques qui les contiennent.

Les composés de la présente invention peuvent être utilisés dans le traitement des maladies qui résultent de troubles dont la cause se situe au niveau du système sérotoninergique central et qui sont connues pour être médiées au niveau de la recapture de sérotonine et/ou au niveau des récepteurs 5-$HT_{1A}$ telles que l'anxiété, les attaques de panique, les troubles obsessionnels compulsifs, les troubles impulsifs, les troubles cognitifs, les phobies et la dépression.

En ce qui concerne le traitement de la dépression, les inhibiteurs sélectifs de recapture de sérotonine (SSRI) représentent actuellement l'une des classes de médicaments les plus efficaces. Toutefois, leurs effets thérapeutiques bénéfiques n'apparaissent, au minimum, pas avant la fin de la deuxième semaine de traitement, et la plupart du temps, au cours de la troisième, voire de la quatrième semaine. Cet inconvénient majeur nuit à l'efficacité de cette classe de produits. Ce temps de latence peut être expliqué par la désensibilisation des récepteurs 5-$HT_{1A}$ des corps cellulaires. En effet, il a été montré (*TIPS,* **1993**, 14, 262) que l'efficacité d'un SSRI tel que la fluoxétine pouvait être réduite par l'activation des récepteurs 5-$HT_{1A}$ avec comme résultante, la réduction de la fréquence de décharge des neurones sérotoninergiques.

En conséquence, un blocage de ces récepteurs 5-$HT_{1A}$ pourrait conduire à une thérapie plus efficace (en diminuant le temps de latence). Récemment, une étude clinique conduite avec un agoniste partiel 5-$HT_{1A}$ (*J. Clin. Psychopharmacol.*, **1995**, 15, 217) montre qu'une telle substance peut améliorer l'efficacité et/ou conduire à diminuer la durée de mise en action d'un SSRI administré de façon concomitante.

Des expériences in-vitro et in-vivo ont permis de démontrer que les composés de la présente invention associent une activité de type inhibitrice sélective de la recapture de sérotonine (SSRI) et une activité antagoniste ou agoniste partielle au niveau des récepteurs 5-$HT_{1A}$.

Ainsi, grâce à leur activité pharmacologique spécifique, les composés de la présente ' invention, outre le fait qu'ils soient nouveaux, peuvent être utiles pour le traitement de l'anxiété, de la dépression, des attaques de panique, des troubles obsessionnels compulsifs, des phobies, des troubles impulsifs et des troubles cognitifs.

Des composés de structures proches ont été décrits dans la littérature. C'est le cas plus particulièrement des demandes de brevet FR 2 738 822, FR 2 761 071 et FR 2 738 823 qui revendiquent notamment des composés possédant un motif 4(1-pipérazinyl)thiéno[3,2-c]pyridine. Ces composés sont utiles dans le traitement de l'hypertension, de l'insuffisance cardiaque, de l'arthrite ou des désordres microcirculatoires. Le brevet US 4,677,104 revendique quant à lui des composés présentant soit un motif 4(1-pipérazinyl)thiéno, ou furo, ou 1*H*-pyrrolo[3,2-c]pyridine, soit un motif 7(1-pipérazinyl)thiéno, ou furo, ou 1*H*-pyrrolo[2,3-c]pyridine, ceux-ci étant utiles en tant qu'agents antipsychotiques ou anxiolitiques. Ces composés se distinguent largement de ceux décrits dans la présente invention du fait de la présence d'un système cyclique très différent substitué sur le motif pipérazinyl.

Plus particulièrement, la présente invention concerne les composés de formule (I) :

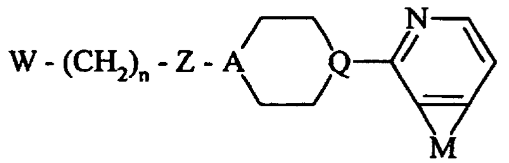

(I)

dans laquelle :

W représente :

- soit un groupement naphtyle éventuellement substitué, par un ou plusieurs groupements, identiques ou différents, choisis parmi halogène, alkyle ($C_1$-$C_6$) linéaire ou ramifié, hydroxy, alkoxy ($C_1$-$C_6$) linéaire ou ramifié, cyano, nitro, trihalogénoalkyle ($C_1$-$C_6$) linéaire ou ramifié, méthylènedioxy et éthylènedioxy,
- soit un groupement de formule Y telle que :

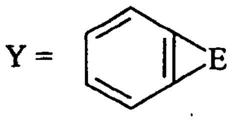

dans laquelle E représente, avec les atomes de carbone du cycle phényle auxquels il est lié, un système monocyclique, insaturé, partiellement saturé, ou aromatique, de 5 à 7 chaînons, comportant au moins un hétéroatome choisi parmi oxygène, azote et soufre, ledit groupement Y pouvant être lié au groupement $(CH_2)_n$ des composés de formule (I), soit par la partie phényle soit par le système monocyclique E, et chacun desdits groupements Y pouvant être éventuellement substitués par un ou plusieurs groupements, identiques ou différents, choisis parmi halogène, hydroxy, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, hétérocycloalkylalkylène ($C_1$-$C_6$) linéaire ou ramifié (dans lequel l'hétérocycle est un système monocyclique insaturé ou aromatique, de 5 à 6 chaînons, contenant de 1 à 4 hétéroatomes choisis parmi azote, soufre, et oxygène), et oxo,
étant entendu que dans ce cas, le groupement Y ne peut être substitué que par un seul groupement oxo et que E représente alors, avec les atomes de carbone du cycle phényle auxquels il est lié, un monocycle à 5 chaînons contenant deux hétéroatomes, identiques ou différents, choisis parmi oxygène et azote,

n représente un entier compris entre 1 et 6 inclus,

Z représente une liaison simple, un atome d'oxygène ou un atome d'azote substitué par un groupement choisi parmi hydrogène, alkyle ($C_1$-$C_6$) linéaire ou ramifié (lui-même éventuellement substitué par un ou plusieurs groupements hydroxy), et arylalkyle ($C_1$-$C_6$) linéaire ou ramifié,

A représente un groupement CH ou un atome d'azote,

Q représente un groupement CH ou un atome d'azote,
étant entendu qu'au moins l'un des groupements A ou Q représente un atome d'azote, et que A représente un atome d'azote quand Z représente une liaison simple,

M représente, avec les atomes de carbone du cycle pyridinyle auxquels il est lié, un groupement thiéno, furo, pyrrolo ou oxopyrrolo,

leurs isomères ainsi que leurs sels d'addition à un acide ou à une base pharmaceutiquement acceptable.

Par groupement aryle, on entend un groupement phényle, naphtyle, dihydronaphtyle ou tétrahydronaphtyle.

D'une façon avantageuse, les substituants W préférés de l'invention sont les groupements naphtyle, 2,3-dihydro-1,4-benzodioxinyle, chromanyle, 2*H*-chroményle, isochromanyle, 2,3-dihydrobenzofuranyle, benzofuranyle, 1,3-dihydro-2*H*-benzimidazol-2-one, 1,3-benzoxazol-2-one, et indolyle.

Dans le cas où W est éventuellement substitué par un groupement hétérocycloalkylalkylène ($C_1$-$C_6$) linéaire ou ramifié, alors ledit groupement hétérocycloalkylalkylène représente avantageusement un groupement hétérocycloalkylméthylène dans lequel l'hétérocycloalkyle est un système monocyclique insaturé ou aromatique, à 5 chaînons contenant de 1 à 4 atomes d'azote. D'une façon préférentielle, ledit groupement hétérocycloalkylméthylène représente un groupement 1,2,4-triazol-1-ylméthyle, imidazol-1-ylméthyle ou 1*H*-imidazole-5-ylméthyle.

D'une façon particulièrement avantageuse, les substituants W préférés selon l'invention sont les groupements 1-naphtyl, isochroman-1-yl et 2,3-dihydro-1-benzofuran-5-yl.

D'une autre façon particulièrement avantageuse, les substituants W préférés selon l'invention sont l'invention sont le groupement 5-(1,2,4-triazol-1-ylméthyl)-1*H*-indol-3-yl, le groupement 5-(imidazol-1-ylméthyl)-1*H*-indol-3-yl, et le groupement 5-(1*H*-imidazol-5-ylméthyl)-1*H*-indol-3-yl.

D'une troisième façon particulièrement avantageuse, les substituants W préférés selon l'invention sont les groupements 1,3-benzoxazol-2-one-1-yl et 1,3-dihydro-2*H*-benzimidazole-2-one-1-yl.

D'une façon préférentielle, Z représente une liaison simple dans les composés de l'invention.

Selon une variante intéressante, les composés préférés de l'invention sont les composés de formule (I) dans laquelle Z représente un atome d'azote substitué par un groupement choisi parmi hydrogène, méthyle ou hydroxyéthylène lorsque A représente un groupement CH, Q représente un atome d'azote, n prend la valeur 1 et W représente un groupement 2-naphtyle.

Selon une variante avantageuse de l'invention, les composés préférés sont ceux pour lesquels n représente

2 ou 3.

Selon une autre variante avantageuse de l'invention, les composés préférés sont ceux pour lesquels M représente, avec les atomes de carbone du cycle pyridinyle auxquels il est lié, un groupement thiényle ou furyle.

D'une façon particulièrement avantageuse, les composés préférés de l'invention sont :

- le 4-{1-[2-(1-naphtyl)éthyl]-4-pipéridinyl}thiéno[3,2-c]pyridine,
- le 4-{1-[2-(1-naphtyl)éthyl]-4-pipéridinyl}furo[3,2-c]pyridine,
- le 4- {4-[2-(2,3-dihydrobenzofuran-5-yl)éthyl]-1-pipérazinyl}furo[3,2-c]pyridine,
- le 4-{4-[2-(isochroman-1-yl)éthyl]-1-pipérazinyl}thiéno[3,2-c]pyridine,
- le 4-(4-{2-[5-(1,2,4-triazol-1-ylméthyl)-1*H*-indol-3-yl]éthyl}-1-pipérazinyl)furo[3,2-c] pyridine,
- le 4-(4-{2-[5-(1,2,4-triazol-1-ylméthyl)-1*H*-indol-3-yl]éthyl}-1-pipérazinyl)-1*H*-pyrrolo[3,2-c] pyridine,
- le 1-[2-(4-furo[3,2-c]pyridin-4-yl-1-pipérazinyl)éthyl]-1,3-dihydro-2*H*-benzimidazol-2-one,
- et le 1-[2-(4-thiéno[3,2-c]pyridin-4-yl-1-pipérazinyl)éthyl]-1,3-dihydro-2*H*-benzimidazol-2-one.

Les isomères ainsi que les sels d'addition à un acide ou à une base pharmaceutiquement acceptable, des composés préférés font partie intégrante de l'invention.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique. maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I), caractérisé en ce qu'on utilise comme produit de départ, un composé de formule (II) :

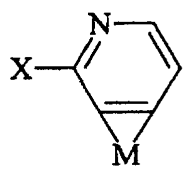

(II)

dans laquelle M est tel que défini dans la formule (I) et X représente un atome de chlore, de brome, d'iode, ou un groupe partant tel que mésyle, tosyle ou triflyle,

* composé de formule (II) que l'on fait réagir en présence d'une base avec un composé de formule (III) :

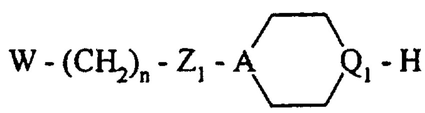

(III)

dans laquelle W, n et A sont tels que définis dans la formule (I), $Q_1$ représente un atome d'azote, et $Z_1$ représente une liaison simple, un atome d'oxygène ou un groupement NH,
pour conduire aux composés de formule (I/a), cas particulier des composés de formule (I) :

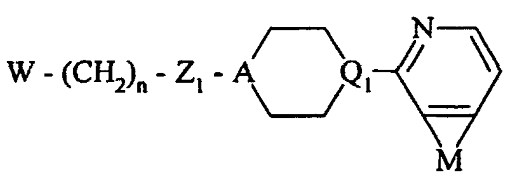

(I/a)

dans laquelle W, n, $Z_1$, A, M et $Q_1$ sont tels que définis précédemment,

* ou composés de formule (II) que l'on transforme, selon des conditions classiques de la synthèse organique, en composés de formule (IV) :

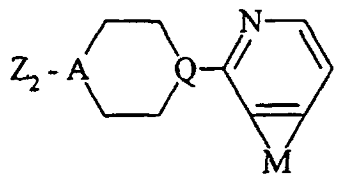

(IV)

dans laquelle A, Q et M sont tels que définis dans la formule (I), et $Z_2$ représente un atome d'hydrogène quand A représente un atome d'azote ou $Z_2$ représente un groupement $NH_2$ quand A représente un groupement CH, composés de formule (IV) que l'on fait réagir ,

- ***soit***, en présence d'un agent de couplage, avec un composé de formule (V) :

$$W - (CH_2)_{n-1} - CO_2H \qquad \textbf{(V)}$$

dans laquelle W et n sont tels que définis dans la formule (I),
pour conduire aux composés de formule (VI) :

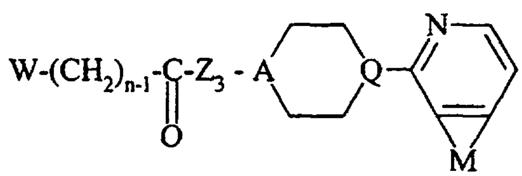

(VI)

dans laquelle W, n, A, Q et M sont tels que définis dans la formule (I), et $Z_3$ représente une liaison quand A représente un atome d'azote ou $Z_3$ représente un groupement NH quand A représente un groupement CH,
composés de formule (VI) que l'on traite par un agent réducteur, selon des conditions classiques de la synthèse organique, pour conduire aux composés de formule (I/b), cas particulier des composés de formule (I) :

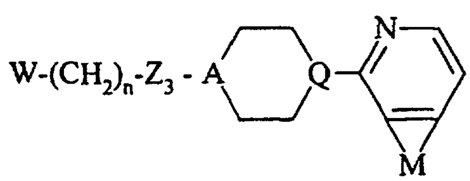

(I/b)

dans laquelle W, n, A, Q, M et $Z_3$ sont tels que définis précédemment,

- ***soit*** avec un composé de formule (VII), dans des conditions d'amination réductive :

$$W - (CH_2)_{n-1} - CHO \qquad \textbf{(VII)}$$

dans laquelle W et n sont tels que définis précédemment,
pour conduire directement aux composés de formule (I/b) telle que définie précédemment,

- ***soit*** avec un composé de formule (VIII) en présence d'un agent de transfert de phase ou d'une base :

$$W - (CH_2)_n - X \ \textbf{(VIII)}$$

dans laquelle W et n sont tels que définis précédemment, et X représente un atome de chlore, de brome, d'iode, ou un groupement libérable tel que tosyle, mésyle ou triflyle,
pour conduire également, directement, aux composés de formule (I/b) telle que définie précédemment,

les composés de formules (I/a) et (I/b) formant les composés de formule (I/c) :

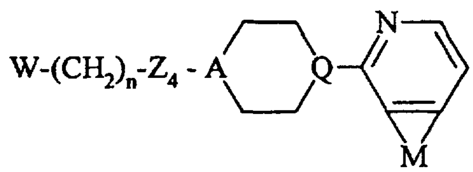

(I/c)

dans laquelle W, n, A, Q et M sont tels que définis dans la formule (I) et $Z_4$ représente une liaison, un atome d'oxygène ou un groupement NH,

composés de formule (I/c), dans le cas particulier ou $Z_4$ prend la valeur $Z'_4$ et représente un groupement NH, et A prend la valeur $A_1$ et représente un groupement CH, que l'on soumet à une réaction d'alkylation selon des méthodes classiques de la synthèse organique, pour conduire aux composés de formule (I/d), cas particulier des composés de formule (I) :

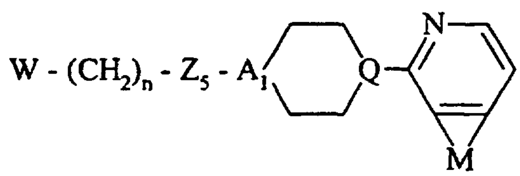

(I/d)

dans laquelle W, n, Q, M et $A_1$ sont tels que définis précédemment et $Z_5$ représente un atome d'azote substitué par un groupement alkyle ($C_1$-$C_6$) linéaire ou ramifié (lui-même éventuellement substitué par un ou plusieurs groupements hydroxy), ou arylalkyle ($C_1$-$C_6$) linéaire ou ramifé,

les composés (I/a) à (I/d) formant l'ensemble des composés de l'invention, que l'on purifie, le cas échéant, selon des techniques classiques de purification, dont on sépare éventuellement les isomères selon une technique classique de séparation, et que l'on transforme, si on le souhaite, en ses sels d'addition à un acide ou à une base pharmaceutiquement acceptable.

les composés de formule (II), (III), (V), (VII) et (VIII) sont soit des composés commerciaux, soit obtenus selon des méthodes classiques de la synthèse organique.

La présente invention a également pour objet les compositions pharmaceutiques renfermant comme principe actif au moins un composé de formule (I), ses isomères ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions selon l'invention, il sera cité plus particulièrement celles qui conviennent pour l'administration orale, parentérale (intraveineuse, intramusculaire, ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

De part l'activité inhibitrice sélective de la recapture de sérotonine et l'activité antagoniste ou agoniste partielle des récepteurs 5-$HT_{1A}$ des composés de l'invention, les compositions pharmaceutiques contenant comme principe actif au moins un composé de l'invention, sont utiles dans le traitement de la dépression, de l'anxiété, des attaques de panique, des troubles obsessionnels compulsifs, des phobies, des troubles impulsifs et des troubles cognitifs.

La posologie utile varie selon l'âge et le poids du patient, la voie d'administration, la nature et la sévérité de l'affection, la prise de traitements associés éventuels, et s'échelonne de 0,5 mg à 50 mg en une ou plusieurs prises par jour.

Les exemples suivants illustrent l'invention mais ne la limitent en aucune façon.

Les produits de départ utilisés sont soit des produits connus, soit préparés selon des modes opératoires connus.

Les différentes préparations conduisent à des intermédiaires de synthèse utiles pour la préparation des composés de l'invention.

Les structures des composés décrits dans les exemples et les préparations ont été déterminées selon les techniques spectrophotométriques usuelles (infrarouge, résonance magnétique nucléaire, spectrométrie de masse...). Les points de fusion ont été déterminés soit à la platine chauffante de Kofler (B.K.), soit à la platine chauffante sous

microscope (M.K.).

***PREPARATION 1 : 4-[(Trifluorométhyl)sulfonyl]furo[3,2-c]pyridine***

A une solution de 14,8 mmol de furo[3,2-c]pyridin-4(5*H*)-one (*J. Med. Chem.*, **1989**, 32, 1147-1156) et 2,1 équivalents de pyridine, dans 80 ml de dichlorométhane, refroidie à -78°C, sont ajoutés goutte à goutte 2,1 équivalents d'anhydride triflique dans 10 ml de dichlorométhane. La réaction est maintenue 1 heure à -78°C puis ramenée 12 heures à température ambiante. Après hydrolyse du milieu réactionnel par addition de 20 ml d'eau, puis extraction au dichlorométhane, la phase organique est séchée, filtrée puis concentrée sous pression réduite. Une chromatographie sur gel de silice (dichlorométhane) permet d'isoler le produit attendu.

***PREPARATION 2 : 4-[(Trifluorométhyl)sulfonyl]thiéno[3,2-c]pyridine***

On procède comme dans la préparation 1 en utilisant comme substrat la thiéno[3,2-c]pyridin-4(5*H*)-one.
**Point de fusion : 110-112°C (B.K.)**

***PREPARATION 3 : 4-(1-Pipérazinyl)furo[3,2-c]pyridine***

Une solution de 0,065 mol de 4-chloro-furo[3,2-c]pyridine et 5 équivalents de pipérazine, dans 25 ml d'éthanol, sous atmosphère inerte est chauffée à 120°C pendant 17 heures. Après refroidissement et concentration sous pression réduite, le résidu obtenu est agité en présence de 150 ml d'eau et 150 ml de dichlorométhane. Après décantation, et extraction au dichlorométhane, les phases organiques sont séchées, filtrées puis concentrées sous pression réduite permettant d'isoler le produit attendu.
**Point de fusion : 90-92°C (B.K.)**

***PREPARATION 4 : 7-(1-Pipérazinyl)thiéno[2,3-c]pyridine***

On procède comme dans la préparation 3 en utilisant comme substrat la 7-chloro-thiéno[2,3-c]pyridine.

***PREPARATION 5 : 7-(1-Pipérazinyl)furo[2,3-c]pyridine***

On procède comme dans la préparation 3 en utilisant comme substrat la 7-chloro-furo[2,3-c] pyridine.
**Point de fusion : 60-65°C (B.K.)**

***PREPARATION 6 : 4-(1-Pipérazinyl)thiéno[3,2-c]pyridine***

On procède comme dans la préparation 3 en utilisant comme substrat la 4-chloro-thiéno[3,2-c]pyridine.
**Point de fusion : 95-100°C (B.K.)**

***PREPARATION 7 : 4-(4-Pipéridinyl)furo[3,2-c]pyridine***

***Stade A : 1-Acétyl-4-hydroxy-4-(tributylstannyl)pipéridine***

A une solution, sous atmosphère inerte et à 0°C, de 0,32 mmol de diisopropylamine dans 750 ml de tétrahydrofurane, est additionné en 5 minutes 0,32 mmol de n-butyllithium (1,6 M dans l'hexane). Après 15 minutes d'agitation, 0,32 mmol d'hydrure de tri-nbutylétain est additionné. Après 1 h 30 à 0°C, le milieu réactionnel est refroidi à -70°C et 0,26 mmol de N-acétyl-pipéridone dans 225 ml de tétrahydrofurane est additionné puis le milieu est dilué par addition de 380 ml de tétrahydrofurane. Après 2 h 30 d'agitation, le milieu réactionnel est hydrolysé par addition de 200 ml d'une solution aqueuse à 10 % de $NaH_2PO_4$, ramené à 0°C, puis dilué par 2 litres d'éther et 1 litre d'eau. Après décantation, lavage par une solution saturée en NaCl, la phase organique est séchée, filtrée puis concentrée sous pression réduite. Une chromatographie sur gel de silice (dichlorométhane) permet d'isoler le produit attendu.

***Stade B : 1-Acétyl-1,2,3,6-tétrahydro-4-tributylstannyl-pyridine***

A une solution à 0°C, sous atmosphère inerte, de 0,16 mmol du composé obtenu dans le stade A et 2,8 équivalents de triéthylamine dans 1 litre de dichlorométhane, sont additionnés goutte à goutte 1,3 équivalents de chlorure de mésyle dans 15 ml de dichlorométhane. Après 4 jours d'agitation à température ambiante, une solution aqueuse saturée en NaCl est additionnée. Après extraction, séchage, filtration et concentration sous pression réduite

de la phase organique, une chromatographie sur gel de silice (dichlorométhane/éthanol : 97/3) du résidu, permet d'isoler le produit attendu.

***Stade C* : 4-(1-Acétyl-1,2,3,6-tétrahydropyridin-4-yl)furo[3,2-c]pyridine**

A une solution de 52,3 mmol du produit de la préparation 1 et 1,2 équivalents du produit obtenu au stade B dans 800 ml de dioxane, sont additionnés, sous flux d'argon, 7 équivalents de LiCl, puis 0,03 équivalents de $Pd(PPh_3)_4$. La réaction est chauffée à 100°C et 0,03 équivalents de $Pd(PPh_3)_4$ sont ajoutés successivement après 12 heures, 24 heures puis 48 heures de réaction, puis le milieu réactionnel est maintenu 24 heures supplémentaires à 100°C. Après refroidissement, filtration sur Célite, et concentration sous pression réduite, une chromatographie sur gel de silice (dichlorométhane/éthanol : 97/3) du résidu permet d'isoler le produit attendu qui est ensuite cristallisé dans le pentane.
**Point de fusion : 128-130°C (B.K.)**

***Stade D* : 4-(1-Acétylpipéridin-4-yl)furo[3,2-c]pyridine**

A une solution de 3,4 g du produit obtenu au stade C et 4,4 g de formiate d'ammonium dans 150 ml de méthanol anhydre, sous courant d'argon, est additionné 0,7 g de Pd/C (10 %). Après 45 minutes de reflux, le milieu réactionnel est refroidi et 4,4 g de formiate d'ammonium et 0,2 g de Pd/C (10 %) sont additionnés puis le milieu réactionnel est porté à reflux pendant 30 minutes. Après retour à température ambiante, filtration sur Célite et concentration sous pression réduite, le résidu est repris à l'eau puis extrait au dichlorométhane. Après extraction, séchage, filtration et concentration sous pression réduite, le produit attendu est isolé.
**Point de fusion : 108-110°C (B.K.)**

***Stade E* : 4-(4-Pipéridinyl)furo[3,2-c]pyridine**

Une solution de 2,7 g du produit obtenu dans le stade D, dans 80 ml d'HCl (4N) est chauffée 12 heures à 100°C, puis refroidie, lavée à l'éther, et basifiée jusqu'à pH = 10 par addition d'une solution aqueuse de soude. Après extraction au dichlorométhane, la phase organique est séchée, filtrée puis évaporée à sec permettant d'isoler le produit attendu.

***PREPARATION 8 : 4-(4-Pipéridinyl)thiéno[3,2-c]pyridine***

***Stade A* : 2-(2-Nitroéthyl)thiophène**

A une solution de 32,2 mmol de 2-(2-nitrovinyl)thiophène dans 210 ml de chloroforme et 70 ml d'isopropanol sont additionnés 15,7 g de silice 60 (230-400 mesh) puis 2,95 g de $NaBH_4$. Après 1 heure d'agitation à température ambiante, 5 ml d'acide acétique sont ajoutés goutte à goutte puis après 15 minutes, la suspension obtenue est filtrée puis rincée au dichlorométhane. Une concentration sous pression réduite du filtrat permet d'isoler le produit attendu.

***Stade B* : 2-(2-Aminoéthyl)thiophène**

A 42 g du produit obtenu au stade A dans 400 ml de méthanol est additionné 0,5 g d'oxyde de platine, puis le milieu réactionnel est placé sous flux d'hydrogène, à température ambiante. Après 6 heures, une filtration sur Célite suivie d'une évaporation permet d'isoler le produit attendu.

***Stade C* : 1-Benzyl-N-[2-(2-thiényl)éthyl]-4-pipéridinecarboxamide**

A une solution de 4,12 g de sodium dans 103 ml d'éthanol anhydre sont ajoutés 22,15 g de 1-benzyl-4-pipéridinecarboxylate d'éthyle et 1 équivalent du produit obtenu au stade B. Après 12 heures de réaction au reflux, le milieu réactionnel est évaporé. Le résidu est repris au dichlorométhane, lavée à l'eau, puis la phase organique est séchée, filtrée et évaporée permettant d'obtenir une huile qui cristallise dans l'éther isopropylique.

***Stade D* : 4-(1-Benzyl-4-pipéridinyl)-6,7-dihydro-thiéno[3,2-c]pyridine**

Une solution de 2 g du produit obtenu au stade C dans 8 ml de toluène, et 12 mmol de $POCl_3$, est portée au reflux. Après 4 heures, le milieu réactionnel est versé sur de la glace, basifié par addition d'une solution de soude à 20 % puis extrait au dichlorométhane. La phase organique est séchée, filtrée puis évaporée permettant d'isoler le produit attendu.

***Stade E* : 4-(1-Benzyl-4-pipéridinyl)thiéno[3,2-c]pyridine**

6,5 g du composé obtenu au stade D et 0,3 g de Pd/C (5 %) sont chauffés à 220-240°C, sous azote, pendant 40 minutes, puis 0,3 g de Pd/C est à nouveau ajouté et la réaction est maintenue pendant 50 minutes. Après retour à température ambiante, reprise à l'éthanol, filtration sur Célite et concentration sous pression réduite, une chromatographie sur gel de silice (dichlorométhane/méthanol : 95/5) du résidu permet d'isoler le produit attendu.

***Stade F* : 4-Thiéno[3,2-c]pyridin-4-yl-1-pipéridinecarboxylate d'éthyle**

A une suspension de 93 ml de chloroformiate d'éthyle, 0,89 g de $K_2CO_3$ dans 5 ml de toluène est additionné goutte à goutte 1 g du produit obtenu au stade E. Après 12 heures à température ambiante, le milieu réactionnel est dilué à l'eau, extrait au toluène puis concentré sous pression réduite. Une chromatographie sur gel de silice (dichlorométhane/méthanol : 95/5) permet d'isoler le produit attendu.

***Stade G* : 4-(4-Pipéridinyl)thiéno[3,2-c]pyridine**

Une solution de 0,57 g du produit obtenu au stade F dans 4 ml d'HCl (5N) est portée au reflux pendant 12 heures, puis refroidie, lavée à l'éther, basifiée par addition de soude à 20 %, et extraite au dichlorométhane. Après séchage, filtration et concentration sous pression réduite de la phase organique, une chromatographie sur gel de silice ($CH_2Cl_2$/MeOH/$NH_4OH$ : 90/10/1) permet d'isoler le produit attendu.

***PREPARATION 9 : Chlorhydrate de 4-(4-aminopipéridin-1-yl)thiéno[3,2-c]pyridine***

***Stade A* : tert-Butyl-4-pipéridinylcarbamate**

A une solution de 0,224 mol de *tert*-butyl-1-benzyl-4-pipéridinylcarbamate dans 800 ml d'éthanol anhydre, sont additionnés 6 g de Pd/C (10 %). La réaction est placée sous 4 bars de pression d'hydrogène, à 50°C pendant 48 heures, puis filtrée sur Célite et concentrée sous pression réduite permettant d'isoler le produit attendu.
**Point de fusion : 154-156°C (B.K.)**

***Stade B* : tert-Butyl-1-thiéno[3,2-c]pyridin-4-yl-pipéridinylcarbamate**

Une solution de 5,65 g du produit obtenu dans le stade A et 1 équivalent du produit de la préparation 2 dans du DMSO est chauffée 2 heures à 50°C, puis jetée sur de la glace et extraite à l'acétate d'éthyle. Après lavage à l'eau, la phase organique est séchée, filtrée puis évaporée. Une chromatographie sur gel de silice (dichlorométhane/méthanol : 90/10) permet d'isoler le produit attendu.

***Stade C* : Chlorhydrate de 4-(4-aminopipéridin-1-yl)thiéno[3,2-c]pyridine**

Un courant d'acide chlorhydrique gazeux est injecté dans une solution de 5,4 g du produit obtenu au stade B dans 200 ml d'acétate d'éthyle, et la température est élevée pendant quelques minutes à 50°C. Après retour à température ambiante et agitation pendant 2 heures, le précipité obtenu est filtré et rincé à l'acétate d'éthyle permettant d'isoler le produit attendu.
**Point de fusion : > 260°C (B.K.)**

***PREPARATION 10 : 7-(1-Pipérazinyl)-1,3-dihydro-2H-pyrrolo[2,3-c]pyridine-2-one***

***Stade A* : [2-(4-Benzyl-1-pipérazinyl)-3-nitro-4-pyridinyl]acétonitrile**

Une solution de 15 g de (2-chloro-3-nitro-pyridin-4-yl)acétonitrile et 1,1 équivalents de N-benzyl-pipérazine est chauffée à 80°C, sous flux d'argon pendant 10 minutes, puis concentrée sous pression réduite. Une chromatographie sur gel de silice (dichlorométhane/méthanol : 100/0 à 95/5) permet d'isoler le produit attendu.

***Stade B* : [2-(4-Benzyl-1-pipérazinyl)-3-amino-4-pyridinyl]acétonitrile**

A une suspension de 59 mmol du produit obtenu au stade A dans 1,5 l d'éthanol absolu, portée à 80°C pendant 1 heure, sont additionnés 43 ml d'acide chlorhydrique et 35 g de poudre de fer. Après 2 heures 30 à 80°C, filtration à chaud, et concentration sous pression réduite du milieu réactionnel, le résidu est repris à l'eau, basifié par

une solution de soude à 20 %, extrait à l'acétate d'éthyle, puis la phase organique est alors séchée, filtrée, évaporée et une chromatographie sur gel de silice (dichlorométhane/éthanol : 90/10) du résidu permet d'isoler le produit attendu.

***Stade C** : 7-(4-Benzyl-1-pipérazinyl)-1,3-dihydro-2H-pyrrolo[2,3-c]pyridine-2-one*

Une suspension de 9,2 g du composé obtenu au stade B précédent dans 300 ml d'une solution aqueuse d'acide chlorhydrique 6N est chauffée à reflux pendant 4 heures. Après concentration sous pression réduite, le résidu solide est dissous par de l'eau et amené à pH basique par de la soude à 20 %. La solution est extraite par de l'acétate d'éthyle, puis les phases organiques sont séchées sur sulfate de magnésium et concentrées sous pression réduite pour donner le produit attendu.
**Point de fusion : 218-220°C (M.K.)**

***Stade D** : 7-(1-Pipérazinyl)-1,3-dihydro-2H-pyrrolo[2,3-c]pyridine-2-one*

Une suspension de 1,9 g du composé obtenu au stade C dans de 250 ml d'éthanol absolu est hydrogénée en présence de palladium sur charbon à 10 % (0,3 équivalent) sous 1 bar à 50°C pendant 2 jours. Le catalyseur est éliminé par filtration, l'évaporation du solvant sous pression réduite conduit après purification à l'obtention du produit attendu.
**Point de fusion : 200-204°C (M.K.)**

***PREPARATION 11 : 7-(4-Amino-1-pipéridinyl)-1,3-dihydro-2H-pyrrolo[2,3-c]pyridine-2-one***

***Stade A** : {2-[4-(tert-Butoxycarbonylamino)-1-pipéridinyl]-3-nitro-4-pyridinyl}acétonitrile*

On procède comme dans le stade A de la préparation 10 en utilisant comme substrat la 4-(*tert*-butoxycarbonylamino)pipéridine.
**Point de fusion : 160-170°C**

***Stade B** : {2-[4-(tert-Butoxycarbonylamino)-1-pipéridinyl]-3-amino-4-pyridinyl}acétonitrile*

1,9 g du produit obtenu au stade A précédent sont dissous dans 400 ml de méthanol puis hydrogénés en présence de palladium sur charbon à 5 % pendant 8 heures à température ambiante. Après filtration et évaporation, on obtient quantitativement le produit attendu.

***Stade C** : 7-(4-Amino-1-pipéridinyl)-1,3-dihydro-2H-pyrrolo[2,3-c]pyridine-2-one*

On procède comme au stade C de la préparation 10 mais en utilisant le produit obtenu au stade B ci-dessus.

***PREPARATION 12 : 7-(1-Pipérazinyl)-1H-pyrrolo[2,3-c]pyridine***

9,2 g du composé obtenu au stade A de la préparation 10, dans 250 ml d'éthanol absolu, sont hydrogénés en présence de palladium sur charbon à 5 %, sous 3 bars, à 65°C pendant 4 jours. Après filtration du catalyseur et évaporation du solvant, le résidu est purifié par chromatographie sur gel de silice (dichlorométhane/éthanol : 100/0 à 95/0) pour conduire au produit attendu.

***PREPARATION 13 : 7-(4-Amino-1-pipéridinyl)-1H-pyrrolo[2,3-c]pyridine***

***Stade A** : 7-[4-(tert-Butoxycarbonylamino)-1-pipéridinyl]1H-pyrrolo[2,3-c]pyridine*

On procède comme dans la préparation 12 mais en utilisant comme substrat le composé obtenu au stade A de la préparation 11.

***Stade B** : 7-(4-Amino-1-pipéridinyl)-1H-pyrrolo[2,3-c]pyridine*

A une solution de 5,1 g du produit obtenu au stade A ci-dessus dans 100 ml d'éthanol absolu, est ajoutée une solution de 50 ml d'éthanol chlorhydrique 2,5N. La solution résultante est agitée à température ambiante pendant 16 heures. Après concentration sous pression réduite, on dilue à l'eau, alcalinise par de la soude à 20 %, extrait au dichlorométhane, sèche les phases organiques sur sulfate de magnésium et concentre sous pression réduite pour

conduire au produit attendu.

***PREPARATION 14 : 4-(1-Pipérazinyl)-1H-pyrrolo[3,2-c]pyridine chlorhydrate***

***Stade 1 : 1-Benzyl-4-bromo-1H-pyrrolo[3,2-c]pyridine***

A une solution de 52 g de triphénylphosphine dans 1,5 l de dioxane sont ajoutés 35 g de N-bromosuccinimide. Après 1 heure d'agitation, 9 g de 1-benzyl-1,5-dihydro-pyrrolo[3,2-c]pyridin-4-one dans 200 ml de dioxane sont additionnés et le mélange est porté à reflux pendant 15 heures. Après élimination du dioxane sous pression réduite, 100 ml de triéthylamine sont ajoutés au résidu et l'ensemble est alors concentré sous vide. Une chromatographie sur gel de silice (cyclohexane/acétate d'éthyle : 50/50) permet d'isoler le produit attendu.
**Point de fusion : 95-100°C**

***Stade 2 : 1-Benzyl-4-(1-pypérazinyl)-1H-pyrrolo[3,2-c]pyridine***

Une solution de 4,5 g de 1-benzyl-4-bromo-1H-pyrrolo[3,2-c]pyridine dans 25 ml d'éthanol anhydre et 8 g de pipérazine est agitée, sous atmosphère inerte, pendant 15 heures à 120°C, puis ramenée à température ambiante et concentrée sous pression réduite. Le résidu est repris par 200 ml de dichlorométhane et lavé à l'eau. La phase organique est traitée de façon classique puis concentrée permettant d'isoler le produit attendu.

***Stade 3 : 4-(1-Pipérazinyl)-1H-pyrrolo[3,2-c]pyridine et son dichlorhydrate***

A 190 ml d'ammoniac liquide à -75°C sont ajoutés successivement 4,1 g du composé obtenu au stade 2 en solution dans 75 ml de tétrahydrofurane et 1 g de sodium. Après 35 minutes, 2,3 g de chlorure d'ammonium sont ajoutés et on laisse évaporer l'ammoniac. 20 ml d'eau sont alors additionnés et après 30 minutes d'agitation, la solution est extraite à l'éther éthylique, séchée, filtrée et concentrée sous vide. Le résidu est repris par 25 ml d'éthanol et 10 ml d'éther chlorhydrique 2,9 N sont ajoutés, puis le milieu est concentré sous vide. Le solide obtenu est agité dans 30 ml d'acétate d'éthyle pendant 18 heures, ce qui conduit à la formation d'un précipité dont la filtration et le lavage par de l'acétate d'éthyle permettent d'isoler le produit attendu.
**Point de fusion : 320-325°C**

***PREPARATION 15 : 4-(4-Pipéridinyl)-1H-pyrrolo[3,2-c]pyridine***

***Stade 1 : 4-Hydroxy-1-pipéridinecarboxylate de tert-butyle***

A une solution de 25 g de 4-hydroxypipéridine et 39 ml de triéthylamine dans 500 ml de dichlorométhane refroidie à -10°C sont ajoutés 59 g de ditertbutyldicarbonate, en maintenant une température inférieure à 0°C. Le mélange est agité 1 h 30 à une température inférieure à 15°C puis concentré sous vide. Le résidu est repris par 500 ml de dichlorométhane et lavé par 200 ml d'une solution aqueuse d'HCl 1N. Après traitement classique et concentration sous pression réduite, le produit attendu est isolé.
**Point de fusion : 55-60°C**

***Stade 2 : 4-Iodo-1-pipéridinecarboxylate de tert-butyle***

A une suspension de 89 g de triphénylphosphine, 22 g d'imidazole dans 800 ml d'acétonitrile sont ajoutés, à température ambiante, 86 g d'iode. La réaction est exothermique. Après retour à température ambiante, 45,9 g du produit obtenu au stade 1 dans 250 ml d'acétonitrile sont additionnés en 30 minutes. Après 18 heures d'agitation à température ambiante, le milieu réactionnel est filtré, rincé par 200 ml d'acétonitrile et le filtrat résultant est concentré sous vide. Le résidu obtenu est agité dans 250 ml d'éther éthylique pendant 1 heure et le précipité est filtré. Après concentration sous vide du filtrat, une chromatographie sur gel de silice (dichlorométhane/acétate d'éthyle : 90/10) permet d'isoler le produit attendu.

***Stade 3 : 4-(1-Benzyl-1H-pyrrolo[3,2-c]pyridin-4-yl)-1-pipéridinecarboxylate de tert-butyle***

A une suspension de 1,35 g de zinc en poudre dans 10 ml de tétrahydrofurane est ajouté, à température ambiante et sous argon, 0,16 ml de 1,2-dibromoéthane. Le mélange est chauffé 5 minutes à 65°C, puis ramené à température ambiante et 0,23 ml de triméthylsilyle est alors ajouté. Après 30 minutes d'agitation, une solution de 4,5 g du composé obtenu au stade 2 dans 8 ml de tétrahydrofurane est additionnée lentement, puis après 45 minutes une

solution préparée *ex situ* 10 minutes auparavant de 0,2 g de $Pd_2(dba)_3$ et 0,2 g de $P(2\text{-furyl})_3$ dans 5 ml de tétrahydrofurane est joutée par cannulation sous argon, et enfin 5,5 g de 1-benzyl-4-bromo-1*H*-pyrrolo[3,2-c]pyridine dans 30 ml d'un mélange 1/1 tétrahydrofurane/diméthylamine sont additionnés. Le milieu réactionnel est chauffé 16 heures à 65°C, puis après retour à température ambiante, filtré et concentré sous vide. Une chromatographie sur gel de silice (cyclohexane/acétate d'éthyle : 50/50) permet d'isoler le produit attendu.

***Stade 4** : 1-Benzyl-4-(4-pipéridinyl)-1H-pyrrolo[3,2-c]pyridine*

A une solution de 0,9 g du composé obtenu au stade 3 dans 15 ml d'éthanol sont ajoutés 15 ml d'une solution d'éthanol chlorhydrique 2,9 N. Le milieu réactionnel est agité 24 heures à température ambiante puis concentré sous vide permettant d'isoler le produit attendu sous forme de dichlorhydrate. Celui-ci est transformé en base libre par extraction dans le dichlorométhane en présence d'eau préalablement amenée à pH basique par de la soude à 20 %.

***Stade 5** : 4-(4-Pipéridinyl)-1H-pyrrolo[3,2-c]pyridine*

On procède comme au stade 3 de la préparation 14 en utilisant comme substrat le produit obtenu au stade 4 précédent.

***EXEMPLE 1 : 1-Furo[2,3-c]pyridin-7-yl-N-(2-naphtylméthyl)-4-pipéridinamine et son fumarate***

2,5 g de 4-(napht-2-yl méthylamino)pipéridine et 1,6 g de 7-chloro-furo[2,3-c] pyridine sont dissout dans 10 ml d'éthanol contenant 3,6 ml de N,N-diisopropyléthylamine, chauffés à reflux puis évaporés à sec et chromatographiés sur gel de silice (dichlorométhane/éthanol : 95/5). On obtient ainsi une huile qui cristallise, que l'on transforme en fumarate par addition d'une solution d'acide fumarique à 2 % dans l'éthanol.
**Point de fusion : 203-207°C (M.K.) (fumarate)**

***EXEMPLE 2 : 7-[4-(2-Naphtylméthoxy)-1-pipéridinyl]furo[2,3-c]pyridine***

On procède comme dans l'exemple 1 en utilisant comme substrat la 4-(2-naphtylméthoxy)pipéridine.
**Point de fusion : 74-77°C (M.K.)**

***EXEMPLE 3 : 1-Furo[2,3-c]pyridin-7-yl-N-méthyl-N-(2-naphtylméthyl)-4-pipéridinamine***

***Stade A** : N-(2-Naphtylméthyl)-N-[1-(furo[2,3-c]pyridin-7-yl)pipéridin-4-yl] carbamate d'éthyle*

A une solution de 0,9 g du composé de l'exemple 1 dans 50 ml de dichlorométhane et 0,85 ml de triéthylamine est additionnée à 0°C 0,3 ml de chloroformate d'éthyle dans 20 ml de dichlorométhane. Après 48 heures à température ambiante, dilution au dichlorométhane et à l'eau, extraction, séchage et concentration sous pression réduite, une chromatographie sur gel de silice (dichlorométhane/acétate d'éthyle : 95/5) permet d'isoler le produit attendu.
**Point de fusion : 118-120°C**

***Stade B** : 1-Furo[2,3-c]pyridin-7-yl-N-méthyl-N-(2-naphtylméthyl)-4-pipéridinamine*

Une solution de 700 mg de produit obtenu au stade A dans 20 ml de tétrahydrofurane est additionnée à 0°C sur une suspension de 123 mg de $LiAlH_4$ dans 20 ml de tétrahydrofurane. On agite 3 heures à température ambiante puis hydrolyse par 0,17 ml d'eau, 0,5 ml de soude à 20 % et 0,70 ml d'eau. On filtre, évapore et purifie par chromatographie sur gel de silice (dichlorométhane/éthanol : 95/5) permettant d'isoler le produit attendu.
**Point de fusion : 125-127°C (M.K.)**

***EXEMPLE 4 : Fumarate de N-(2-naphtylméthyl)-1-thiéno[2,3-c]pyridin-7-yl-4-pipéridinamine***

On procède comme dans l'exemple 1 en utilisant comme substrat la 7-chloro-thiéno[2,3-c] pyridine.
**Point de fusion : 186-192°C (M.K.)**

***EXEMPLE 5 : Hemi-fumarate de N-(2-naphtylméthyl)-1-furo[3,2-c]pyridin-4-yl-4-pipéridinamine***

On procède comme dans l'exemple 1 en utilisant comme substrat la 4-chloro-furo[3,2-c] pyridine.
**Point de fusion : 194-197°C (M.K.)**

***EXEMPLE 6 : Hemi-fumarate de N-(2-naphtylméthyl)-1-thiéno[3,2-c]pyridin-4-yl-4-pipéridinamine***

On procède comme dans l'exemple 1 en utilisant comme substrat la 4-chloro-thiéno[3,2-c] pyridine.
**Point de fusion : 208-212°C (M.K.)**

***EXEMPLE 7 : 4-{1-[2-(1-Naphtyl)éthyl]-4-pipéridinyl}furo[3,2-c]pyridine et son fumarate***

***Stade A : 1-(4-Furo[3,2-c]pyridine-4-yl-1-pipéridinyl)-2-(1-naphtyl)-1-éthanone***

A une solution de 0,92 g d'acide 1-naphtyl acétique dans 30 ml de dichlorométhane sont ajoutées 5,4 mmoles de carbonyldiimidazole. Après 2 heures à température ambiante, 1 g du composé de la préparation 7 dans 20 ml de dichlorométhane est additionné. Après hydrolyse, extraction, séchage et concentration sous pression réduite, le produit attendu est isolé.
**Point de fusion : 50°C (B.K.)**

***Stade B : 4-{1-[2-(1-Naphtyl)éthyl]-4-pipéridinyl}furo[3,2-c]pyridine et son fumarate***

Une solution de 1,4 g de produit obtenu au stade A précédent dans 50 ml de tétrahydrofurane est introduite sur une suspension de 215 mg de $LiAlH_4$ dans 20 ml de tétrahydrofurane. Après 2 heures à température ambiante, on hydrolyse par 2 ml d'eau, dissout dans 10 ml de tétrahydrofurane, filtre l'insoluble, évapore le filtrat, reprend à l'éther, lave à l'eau, sèche sur MgSO4, filtre et évapore, permettant d'isoler le produit attendu qui est transformé en fumarate.
**Point de fusion (fumarate) : 224-226°C (M.K.)**

***EXEMPLE 8 : 4-{1-[2-(2-Naphtyl)éthyl]-4-pipéridinyl}furo[3,2-c]pyridine***

On procède comme dans l'exemple 7 des stades A à B, en utilisant au stade A l'acide 2-naphtyl-acétique.
**Point de fusion : 96-97°C (M.K.)**

***EXEMPLE 9 : 4-{4-[2-(Benzofuran-2-yl)éthyl]-1-pipérazinyl}furo[3,2-c]pyridine***

On procède comme dans l'exemple 7 des stades A à B en utilisant comme substrats au stade A l'acide 2-benzofuranyl acétique et le produit obtenu dans la préparation 3.
**Point de fusion : 99-100°C (M.K.)**

***EXEMPLE 10 : 4-{4-[2-(1H-Indol-5-yl)éthyl]-1-pipérazinyl}furo[3,2-c]pyridine***

On procède comme dans l'exemple 7 des stades A à B en utilisant comme substrats au stade A l'acide 1*H*-indol-5-yl acétique et le produit obtenu dans la préparation 3.
**Point de fusion : 153-156°C (M.K.)**

***EXEMPLE 11 : 4-{4-[2-(2,3-Dihydrobenzofuran-5-yl)éthyl]-1-pipérazinyl} furo[3,2-c] pyridine***

On procède comme dans l'exemple 7 des stades A à B, en utilisant comme substrats au stade A l'acide 2,3-dihydro-1-benzofuran-5-yl acétique et le produit de la préparation 3.
**Point de fusion : 105-107°C**

***EXEMPLE 12 : 7-{4-[2-(Benzofuran-2-yl]éthyl]-1-pipérazinyl}furo[2,3-c]pyridine***

On procède comme dans l'exemple 7 des stades A à B en utilisant comme substrats au stade A l'acide benzofuran-2-yl acétique et le produit obtenu à la préparation 5.
**Point de fusion : 111-113°C (M.K.)**

***EXEMPLE 13 : 7-{4-[2-(Benzofuran-2-yl)éthyl]-1-pipérazinyl}thiéno[2,3-c]pyridine***

On procède comme dans l'exemple 7 des stades A à B en utilisant comme substrats au stade A l'acide benzofuran-2-yl acétique et le produit obtenu à la préparation 4.
**Point de fusion : 97-98°C (M.K.)**

***EXEMPLE 14 : 4-{1-[2-(1-Naphtyl)éthyl]-4-pipéridinyl}thiéno[3,2-c]pyridine et son chlorhydrate***

On procède comme dans l'exemple 7 des stades A à B en utilisant comme substrat au stade A le produit de la préparation 8.
**Point de fusion (dichlorhydrate) : 256-260°C (M.K.)**

***EXEMPLE 15 : 4-{4-[2-(Benzofuran-2-yl)éthyl]-1-pipérazinyl}thiéno[3,2-c]pyridine***

On procède comme dans l'exemple 7 des stades A à B en utilisant comme substrats au stade A l'acide benzofuran-2-yl acétique et le produit obtenu à la préparation 6.
**Point de fusion : 99-100°C (M.K.)**

***EXEMPLE 16 : 4-{4-[2-(2,3-Dihydro-1,4-benzodioxin-2-yl)éthyl]-1-pipérazinyl}thiéno [3,2-c]pyridine et son chlorhydrate***

On procède comme dans l'exemple 7 des stades A à B en utilisant comme substrats au stade A l'acide 2,3-dihydro-1,4-benzodioxin-2-yl acétique et le produit obtenu à la préparation 6.
**Point de fusion (dichlorhydrate) : 140-145°C (M.K.)**

***EXEMPLE 17 : 7-[4-(2H-Chromèn-3-ylméthyl)-1-pipérazinyl]-1H-pyrrolo[2,3-c] pyridine***

A une solution de 0,8 g de 2*H*-chromène-3-yl carbaldéhyde, et 1 g du produit obtenu à la préparation 12 dans 100 ml de dichlorométhane sont additionnés à température ambiante 1,45 g de triacétoxyborohydrure de sodium et 0,3 ml d'acide acétique. Après 24 heures, le milieu réactionnel est alcalinisé par addition de soude à 20 %, extrait au dichlorométhane, séché, évaporé, et purifié par chromatographie sur gel de silice (dichlorométhane/éthanol : 95/5) permettant d'isoler le produit attendu.
**Point de fusion : 165-169°C (M.K.)**

***EXEMPLE 18 : 7-[4-(Chroman-4-ylméthyl)-1-pipérazinyl]-1H-pyrrolo[2,3-c]pyridine***

On procède comme dans l'exemple 17 en utilisant comme substrat le chroman-4-yl carbaldéhyde.
**Point de fusion : 228-231°C (M.K.)**

***EXEMPLE 19 : 7-{4-[2-(Chroman-4-yl)éthyl]-1-pipérazinyl}-1H-pyrrolo[2,3-c]pyridine***

On procède comme dans l'exemple 17 en utilisant comme substrat le chroman-4-yl acétaldéhyde.
**Point de fusion : 244-247°C (M.K.)**

***EXEMPLE 20 : 4-{4-[2-(1-Naphtyl)éthyl]-1-pipérazinyl}furo[3,2-c}pyridine***

Une solution de 50 ml d'acétone, 1,5 g de 2-(napht-1-yl) 1-bromoéthane, 1,1 g du produit de la préparation 3 et 1,5 g de carbonate de potassium est portée à reflux pendant 24 heures. On dilue à l'eau, extrait à l'éther, épuise à l'acide chlorhydrique 1N et alcalinise avec de la soude à 10 % la phase aqueuse acide. Après extraction au chlorure de méthylène de la phase basique, on sèche, évapore et cristallise de l'éther permettant d'isoler le produit attendu.
**Point de fusion : 94-98°C (M.K.)**

***EXEMPLE 21 : 4-{4-[2-(2-Naphtyl)éthyl]-1-pipérazinyl}furo[3,2-c]pyridine***

On procède comme dans l'exemple 20 en utilisant comme substrat le 2-(napht-2-yl)1-bromoéthane.
**Point de fusion : 113-115°C (M.K.)**

***EXEMPLE 22 : 4-{4-[3-(Benzofuran-2-yl)propyl]-1-pipérazinyl}furo[3,2-c]pyridine et son dichlorhydrate***

On procède comme dans l'exemple 20 en utilisant comme substrat le 2-(3-bromopropyl)benzofurane.
**Point de fusion (dichlorhydrate) : 194-197°C (M.K.)**

*EXEMPLE 23 : N-(2-Naphtylméthyl)-N-[1-(1H-pyrrolo[2,3-c]pyridin-7-yl)-4-pipéridinyl]amine*

On procède comme dans l'exemple 20 en utilisant comme substrats le 2-(α-bromométhyl)naphtalène et le produit obtenu à la préparation 13.
**Point de fusion : 171-173°C (M.K.)**

*EXEMPLE 24 : 7-{4-[(2-Naphtylméthyl)amino]-1-pipéridinyl}-1,3-dihydro-2H-pyrrolo[2,3-c]pyridin-2-one*

On procède comme dans l'exemple 20 en utilisant comme substrats le 2-(α-bromométhyl)naphtalène et le produit obtenu à la préparation 11.
**Point de fusion : 186-190°C (M.K.)**

*EXEMPLE 25 : 7-{4-[2-(2-Naphtyl)éthyl]-1-pipérazinyl}-1,3-dihydro-2H-pyrrolo[2,3-c] pyridin-2-one*

On procède comme dans l'exemple 20 en utilisant comme substrats le 2-(napht-2-yl)1-bromoéthane et le produit obtenu à la préparation 10.
**Point de fusion : 184-186°C (M.K.)**

*EXEMPLE 26: 7-{4-[2-(2-Naphtyl)éthyl]-1-pipérazinyl}-1H-pyrrolo[2,3-c]pyridine*

On procède comme dans l'exemple 20 en utilisant comme substrats le 2-(napht-2-yl)1-bromoéthane et le produit obtenu à la préparation 12.
**Point de fusion : 149-151°C (M.K.)**

*EXEMPLE 27 : 7-{4-[2-(1-Naphtyl)éthyl]-1-pipérazinyl}-1H-pyrrolo[2,3-c]pyridine*

On procède comme dans l'exemple 20 en utilisant comme substrat le produit obtenu à la préparation 12.
**Point de fusion : 169-172°C (M.K.)**

*EXEMPLE 28 : 3-{2-[4-(1H-Pyrrolo[2,3-c]pyridin-7-yl)-1-pipérazinyl]éthyl}-1,3-benzoxazol-2(3H)-one*

On procède comme dans l'exemple 20 en utilisant comme substrats le 3-(2-bromoéthyl)-1,3-benzoxazol-2 (3H)one et le produit de la préparation 12.
**Point de fusion : 225-227°C (M.K.)**

*EXEMPLE 29 : 7-{4-[2-(2H-Chromèn-3-yl)éthyl]-1-pipérazinyl}-1H-pyrrolo[2,3-c] pyridine*

On procède comme dans l'exemple 20 en utilisant comme substrat le 2-(2*H*-chromèn-3-yl)1-bromoéthane et le produit de la préparation 12.
**Point de fusion : 177-180°C (M.K.)**

*EXEMPLE 30 : 7-{4-[2-(2-Naphtyl)éthyl]-1-pipérazinyl}furo[2,3-c]pyridine et son fumarate*

On procède comme dans l'exemple 20 en utilisant le produit de la préparation 5.
**Point de fusion (fumarate) : 175-181°C (M.K.)**

*EXEMPLE 31 : 7-[4-(2,3-Dihydro-1,4-benzodioxin-2-ylméthyl)-1-pipérazine]thiéno [2,3-c]pyridine et son dichlorhydrate*

On procède comme dans l'exemple 20 en utilisant comme substrats la 2-(α-bromoéthyl)2,3-dihydro-1,4-benzodioxine et le produit de la préparation 4.
**Point de fusion (dichlorhydrate) : 195-200°C (M.K.)**

*EXEMPLE 32 : 4-{1-[2-(1-naphtyl)éthyl]-4-pipéridinyl}-1H-pyrrolo[3,2-c]pyridine et son chlorhydrate*

On procède comme dans l'exemple 20 en utilisant comme substrat le produit de la préparation 15.
**Point de fusion (chlorhydrate) : 180-185°C (M.K.)**

***EXEMPLE 33 : 4-(4-{2-[5-(1,2,4-Triazol-1-ylméthyl)-1H-indol-3-yl]éthyl}-1-pipérazinyl) furo[3,2-c]pyridine et son trichlorhydrate***

On procède comme dans l'exemple 20 en utilisant le 2-[5-(1,2,4-triazol-1-ylméthyl)-1*H*-indol-3-yl]-1-bromoéthane à la place du 2-(napht-1-yl)-1-bromoéthane.
**Point de fusion (trichlorhydrate) : 196-200°C (M.K.)**

***EXEMPLE 34 : 4-(4-{2-[5-(1,2,4-triazol-ylméthyl)-1H-indol-3-yl]éthyl}-1-pipérazinyl)-1H-pyrrolo[3,2-c]pyridine et son dichlorhydrate***

On procède comme dans l'exemple 20 en utilisant comme substrats le produit de la préparation 14 et le 2-[5-(1,2,4-triazol-1-ylméthyl)-1*H*-indol-3-yl]-1-bromoéthane.
**Point de fusion (dichlorhydrate) : 205-210°C (M.K.)**

***EXEMPLE 35 : 1-[2-(4-Furo[3,2-c]pyridin-4-yl-1-pipérazinyl)éthyl]-1,3-dihydro-2H-benzimidazol-2-one***

***Stade A : 1-[2-(4-Furo[3,2-c]pyridiny-4-yl-1-pipérazinyl)éthyl]-3-(1-phénylvinyl)-1,3-dihydro-2H-benzimidazol-2-one***

On procède comme dans l'exemple 20 en utilisant comme substrat la 1-(2-bromoéthyl)-3-(1-phénylvinyl)-1,3-dihydro-2*H*-benzimidazol-2-one.

***Stade B : 1-[2-(4-Furo[3,2-c]pyridin-4-yl-1-pipérazinyl)éthyl]-1,3-dihydro-2H-benzimidazol-2-one***

Un mélange, formé de 1,7 g du produit obtenu au stade A de cet exemple, 5 ml d'acide chlorhydrique concentré et 20 ml d'eau, est porté à 65°C pendant 30 minutes. Après refroidissement, le milieu réactionnel est lavé à l'éther, basifié à pH 9-10 par addition de carbonate de potassium, puis extrait à l'éther. Après séchage et purification par chromatographie sur gel de silice, on obtient le produit attendu.
**Point de fusion : 152-154°C (M.K.)**

***EXEMPLE 36 : 1-[2-(4-Thiéno[3,2-c]pyridin-4-yl-1-pipérazinyl)éthyl]-1,3-dihydro-2H-benzimidazol-2-one***

On procède comme dans l'exemple 35 des stades A à B en utilisant au stade A le produit de la préparation 6.
**Point de fusion : 215-220°C (M.K.)**

***EXEMPLE 37 : 4-{4-[N-(Napht-2-ylméthyl)-N-(2-hydroxyéthyl)amino]-4-pipéridinyl} thiéno[3,2-c]pyridine***

***Stade A : {N-(Napht-2-ylméthyl)-N-[1-(thiéno[3,2-c]pyridine-4-yl)]-4-pipéridinyl} amino acétate d'éthyle***

Un mélange formé de 2,6 g du produit obtenu à l'exemple 6, 1 ml de bromoacétate d'éthyle, 2,3 g de carbonate de potassium et 50 ml d'acétonitrile est agité à température ambiante pendant 12 heures, puis filtré et concentré sous pression réduite, permettant d'obtenir le produit attendu.

***Stade B : 4-{4-[N-(Napht-2-ylméthyl)-N-(2-hydroxyéthyl)amino]-4-pipéridinyl} thiéno[3,2-c]pyridine***

Une solution de 4 g du produit obtenu au stade A dans 100 ml de tétrahydrofurane est coulée à 0°C sur une suspension de 0,43 g d'hydrure de lithium et d'aluminium dans 60 ml de tétrahydrofurane. Après 30 secondes à cette température, on ajoute 0,86 g d'hydrure de lithium et d'aluminium et laisse encore 15 minutes en contact. On hydrolyse alors avec 0,6 ml d'eau puis 0,5 ml de soude à 20 %. On filtre, concentre et purifie par chromatographie sur silice (acétate d'éthyle) pour obtenir le produit attendu.
**Point de fusion : 53-55°C (M.K.)**

***EXEMPLE 38 : 4-{4-[2-(Isochroman-1-yl)éthyl]-1-pipérazinyl}thiéno[3,2-c]pyridine et son dichlorhydrate***

A une solution de 11,2 mM de 2-(isochroman-1-yl)éthanol et 1,24 g de triéthylamine, dans 10 ml de dichlorométhane, sont additionnées à 0°C 12,3 mM de chlorure de mésyle dissous dans 10 ml de dichlorométhane. Après deux heures d'agitation à température ambiante, 16,8 mM du produit de la préparation 6 en suspension dans 30 ml de dichlorométhane sont additionnés, puis le milieu réactionnel est porté à reflux et 35 ml d'éthylène glycol sont ajoutés

tout en distillant le dichlorométhane. Après 3 heures à 80°C, la réaction est maintenue 12 heures à température ambiante. Le milieu réactionnel est ensuite versé sur 200 ml d'eau et extrait à l'acétate d'éthyle. Les phases organiques rassemblées sont lavées à l'eau, séchées, filtrées puis concentrées sous pression réduite. Une chromatographie sur gel de silice (acétate d'éthyle) du résidu permet d'isoler le produit attendu sous forme d'huile qui est transformé en dichlorhydrate par action de l'éther chlorhydrique.
**Point de fusion : 146-148°C (M.K.)**

***EXEMPLE 39 : 4-(4-{2-[5-(Imidazol-1-ylméthyl)1H-indol-3-yl]éthyl}-1-pipérazinyl)-1H-pyrrolo[3,2-c]pyridine***

***Stade 1 : 2-(5-Bromo-1H-indol-3-yl)éthanol***

A une suspension de 0,9 g de $LiAlH_4$ dans 35 ml de tétrahydrofurane refroidie à 0°C est ajoutée, goutte à goutte, une solution de 6 g de N-*tert*-butoxycarbonyl-5-bromo-3-éthoxycarbonylméthyl-indole dans 50 ml de tétrahydrofurane. Après 20 heures d'agitation à température ambiante, le milieu est refroidi à 0°C puis traité successivement par 0,9 ml d'eau, 3,2 ml d'une solution aqueuse de soude à 20 % et 3,6 ml d'eau. Après filtration et concentration sous vide du filtrat, le résidu est chromatographié sur gel de silice (dichlorométhane/acétate d'éthyle : 90/10) permettant d'isoler le produit attendu.
**Point de fusion : 85-89°C**

***Stade 2 : 2-(5-Bromo-1H-indol-3-yl)-1-bromoéthane***

A une solution de 4 g du composé obtenu au stade 1 et 7,5 g de tétrabromocarbone dans 100 ml de dichlorométhane refroidie à 0°C est ajoutée une solution de 11,5 g de triphénylphosphine dans 60 ml de dichlorométhane. Après 48 heures d'agitation à température ambiante, le milieu réactionnel est concentrée sous vide. Le résidu obtenu est chromatographié sur gel de silice (dichlorométhane/cyclohexane : 80/20) permettant d'isoler le produit attendu.
**Point de fusion : 65-70°C**

***Stade 3 : 4-{4-[2-(5-Bromo-1H-indol-3-yl)éthyl]-1-pipérazinyl}-1H-pyrrolo[3,2-c] pyridine***

On procède comme dans l'exemple 20 en utilisant comme substrats le produit obtenu au stade 2 et le produit de la préparation 14.
**Point de fusion : 112-115°C (M.K.)**

***Stade 4 : 3-{2-[4-(1H-Pyrrolo[3,2-c]pyridin-4 yl)-1-pipérazinyl]éthyl}-1H-indol-5-carbaldéhyde***

Une solution à -78°C de 1,5 mmol du composé obtenu au stade 3 dans la tétrahydrofurane est traitée par 9 mmol de *tert*-butyllithium (1,8 M dans l'hexane). Après 1 heure d'agitation, 12 mmol de diméthylformamide sont ajoutées et le milieu est ramené à température ambiante. Après 4 heures de réaction, la solution est hydrolysée par une solution saturée de $NH_4Cl$, puis extraite au dichlorométhane. Après traitement classique de la phase organique, une chromatographie sur gel de silice du résidu permet d'isoler le produit attendu.

***Stade 5 : 4-{4-[2-(5-Hydroxyméthyl-1H-indol-3-yl)éthyl]1-pipérazinyl}-1H-pyrrolo [3,2-c]pyridine***

Une solution de 2 mmol du composé obtenu au stade 4 dans 50 ml d'éthanol est traitée par 2,2 mmol de borohydrure de sodium pendant 24 heures. Le solvant est évaporé sous pression réduite et le résidu obtenu est repris par de l'eau, puis extrait au dichlorométhane. La phase organique est séchée, filtrée puis concentrée sous pression réduite permettant d'obtenir le produit attendu.

***Stade 6 : 4-{4-[2-(5-Bromoéthyl-1H-indol-3-yl)éthyl]-1-pipérazinyl}-1H-pyrrolo[3,2-c] pyridine***

On procède comme au stade 2 de cet exemple en utilisant comme substrat le produit obtenu au stade 5.

***Stade 7 : 4-(4-{2-[5-(Imidazol-1-ylméthyl)1H-indol-3-yl]éthyl}-1-pipérazinyl)-1H-pyrrolo[3,2-c]pyridine***

Une solution de 3,7 mmol du composé obtenu au stade 6 et 4,1 mmol de tritylimidazole dans 50 ml d'acétonitrile est portée à reflux pendant 24 heures puis chauffée à 60°C pendant 24 heures en présence de 50 ml de méthanol. Le mélange réactionnel est alors concentré sous vide. Le résidu est repris par un mélange biphasique 5/1 : dichlorométhane/soude à 20 %. La phase organique est séchée, filtrée et concentrée sous pression réduite permettant d'obtenir

le produit attendu.

***EXEMPLE 40 : 4-(4-{2-[5-(1H-Imidazol-5-ylméthyl)-1H-indol-3-yl]éthyl}-1-pipérazinyl)-1H-pyrrolo[3,2-c]pyridine***

***Stade 1 : (3-{2-[4-(1H-Pyrrolo[3,2-c]pyridin-4-yl)-1-pipérazinyl]éthyl}-1H-indol-5-yl)(1-trityl-1H-imidazol-4-yl) méthanol***

A une solution de 2,0 mmol de 4-iodo-1-tritylimidazole dans le dichlorométhane (0,25 M) est additionnée, à température ambiante, une solution de 2,2 mmol de bromure d'éthyl magnésium 3M, puis après 30 minutes, 2,2 mmol du composé obtenu au stade 4 de l'exemple 39. Après 18 heures d'agitation, le mélange est traité par une solution de $NH_4Cl$ saturée, suivi d'une extraction au dichlorométhane, puis traitement classique de la phase organique. Une chromatographie sur gel de silice permet d'isoler le produit attendu.

***Stade 2 : 4-(4-{2-[5-(1H-Imidazol-5-ylméthyl)-1H-indol-3-yl]éthyl}-1-pipérazinyl)-1H-pyrrolo[3,2-c]pyridine***

Une solution, refroidie à -5°C, de 1,9 mmol du composé obtenu au stade 1 et 9,5 mmol de triéthylsilane dans le dichlorométhane, est traitée par une solution de 19 mmol d'acide trifluoroacétique. Après 18 heures d'agitation à température ambiante, le milieu réactionnel est traité par une solution saturée de $NaHCO_3$, puis la phase organique est traitée par une solution 2N d'HCl. La phase aqueuse est basifiée par de la soude à 20 %, et une extraction par le dichlorométhane permet d'isoler le produit attendu après séchage et concentration de la phase organique.

***ETUDES PHARMACOLOGIQUES DES COMPOSES DE L'INVENTION***

***A. Etude in vitro***

***EXEMPLE 41 : Détermination de l'affinité pour les sites de recapture de la sérotonine***

L'affinité a été déterminée par des expériences de compétition avec la [$^3$H]-paroxétine (NEN, Les Ulis, France). Les membranes sont préparées à partir de cortex frontal de rat et incubées en triple avec 1,0 nM de [$^3$H]-paroxétine et le ligand froid dans un volume final de 0,4 ml, pendant 2 heures à 25°C. Le tampon d'incubation contient 50 nM de TRIS-HCl (pH 7,4), 120 mM de NaCl et 5 mM de KCl. La fixation non-spécifique est déterminée avec 10 μM de citalopram. A la fin de l'incubation, le milieu d'incubation est filtré et lavé trois fois avec 5 ml de tampon refroidi. La radioactivité retenue sur les filtres est déterminée par comptage du liquide de scintillation. Les isothermes de liaison sont analysées par régression non-linéaire pour déterminer les valeurs d'$IC_{50}$. Elles sont converties en constante de dissociation ($K_i$) par l'intermédiaire de l'équation de Cheng-Prusoff :

$$K_I = IC_{50}/(1 + L / K_d)$$

dans laquelle L est la concentration de [$^3$H]-paroxétine et $K_d$ est la constante de dissociation de [$^3$H]-paroxétine pour le site de recapture de la sérotonine (0,13 nM). Les résultats sont exprimés en $pK_i$.
Les composés de la présente invention montrent une très bonne affinité pour les sites de recapture de la sérotonine. A titre d'exemple, le $pK_I$ du composé de l'exemple 7 est supérieur à 7,5.

***EXEMPLE 42 : Détermination de l'affinité pour les récepteurs 5-HT$_{1A}$***

L'affinité a été déterminée par des expériences de compétition avec le [$^3$H]-8-OH-DPAT (NEN, Les Ulis, France). Les membranes préparées à partir de cellules CHO transfectées avec le récepteur 5-$HT_{1A}$ ont été préparées comme décrit dans *Neuropharmacol.,* **1997**, 36, 451-459). Les membranes sont incubées en triple avec 0,4 nM de [$^3$H]-8-OH-DPAT et le ligand froid dans un volume final de 1,0 ml, pendant deux heures et demie à 25°C. Le tampon d'incubation contient 25 mM de HEPES-NaOH (pH 7,4) et 5 mM de $MgCl_2$. La fixation non spécifique est déterminée avec 10 μM de 5-HT. A la fin de l'incubation, le milieu d'incubation est filtré au travers de filtres imprégnés avec 0,1 % de polyéthylénimine et lavés trois fois avec 5 ml de tampon refroidi. La radioactivité retenue sur les filtres est déterminée par comptage du liquide de scintillation. Les isothermes de liaison sont analysées par régression non linéaire pour déterminer les valeurs d'$IC_{50}$. Elles sont converties en constante de dissociation ($K_i$) par l'intermédiaire de l'équation de Cheng-Prusoff :

$$K_i = IC_{50} / \{(L / K_d)-1\}$$

dans laquelle L est la concentration de [$^3$H]-8-OH-DPAT et $K_d$ est la constante de dissociation de [$^3$H]-8-OH-DPAT du récepteur 5-$HT_{1A}$ (0,65 nM). Les résultats sont exprimés en $pK_i$.
A titre d'exemple et pour illustrer l'activité des produits de l'invention, le $pK_i$ du produit de l'exemple 7 est supérieur à 7,5.

***B. Etude in vivo***

***EXEMPLE 43 : Expérience de la microdialyse chez le rat***

Les rats sont anesthésiés au pentobarbital (60 mg/kg i.p.). Ils sont placés dans un appareil stéréotaxique de Kopf et le guide de la canule est implanté dans le cortex frontal cingulé suivant les coordonnées décrites comme suit dans l'atlas de Paxinos et Watson (1982) : AP : + 2,2, L : ± 0,6, DV : -0,2. Les rats sont mis en cage séparément et ne sont utilisés en dialyse que 5 jours plus tard. Le jour de la dialyse la sonde est descendue lentement et maintenue dans sa position. La sonde est perfusée à un débit de 1 µl/min avec une solution de 147,2 mM de NaCl, 4 mM de KCl et 2,3 mM de $CaCl_2$ amené à pH 7,3 avec un tampon phosphate (0,1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane. Des sections d'une épaisseur de 100 µm sont coupées et colorées avec du cresyl violet, ce qui permet la vérification de l'emplacement des sondes. La quantification simultanée de dopamine, norépinéphrine et sérotonine est effectuée de la façon suivante : 20 µl d'échantillons de dialyse sont dilués avec 20 µl de phase mobile ($NaH_2PO_4$ : 75 mM, EDTA : 20 µM, sodium 1-decanesulfonate : 1 mM, méthanol : 17,5 %, triéthylamine : 0,01 %, pH : 5,7) et 33 µl d'échantillons sont analysés par HPLC avec une colonne en phase inverse thermostatée à 45°C et quantifiés par l'intermédiaire d'un détecteur coulométrique. Le potentiel de la première électrode du détecteur est fixé à - 90 mV (réduction) et celui de la seconde à ± 280 mV (oxydation). La phase mobile est injectée avec une pompe Beckman 116 à un débit de 2 ml/mn. Les limites de sensibilité pour la dopamine, la norépinéphrine et la sérotonine sont de 0,55 fmole par échantillon. Tous les produits de l'invention sont injectés par voie sous-cutanée dans un volume de 1,0 ml/kg. Les produits sont dissous dans de l'eau distillée additionnée de quelques gouttes d'acide lactique si nécessaire.

**Résultats :**

A titre d'exemple et pour illustrer l'activité des produits de l'invention, le composé de l'exemple 7, administré à la dose de 10 mg/kg, par voie sous-cutanée, augmente le niveau de sérotonine de : 140,5 ± 15,2 % (% maximal de l'effet par rapport au niveau basal défini comme le 0 %).

***EXEMPLE 44 : Test d'agressivité chez des souris isolées***

Ce test permet d'évaluer l'activité anti-agressive *intraspecies* d'un produit chez des souris qui ont été maintenues en isolement pendant plusieurs mois.

**Animaux :**

Le test utilise des souris mâles CD (Charles River) de poids 22 à 25 g. Dès leur arrivée, les animaux sont isolés dans des cages individuelles opaque noir (23 x 14 x 13 cm) avec un couvercle grillagé et stabulés de façon chronique (6 mois environ) dans la pièce d'expérimentation.

**Sélection des couples de souris :**

La sélection des couples de souris agressives qui seront utilisés de façon chronique dans l'étude, commence après un mois d'isolement des animaux. Une ou deux fois par semaine, on place dans la cage d'une souris (résidente), une souris d'une autre cage (intruse) et l'on observe si les deux animaux s'attaquent (reniflements, poursuites, mordillements, morsures) pendant cet essai. A la fin de l'essai (durée maximale de 10 min.), chaque souris est isolée à nouveau dans sa cage respective. Si il y a eu des attaques, le même couple sera testé à nouveau lors du prochain essai ; si il n'y a pas eu d'attaques, chaque souris de ce couple sera mise en présence d'une autre souris, lors de l'essai suivant. On sélectionne ainsi, au cours d'essais successifs, à raison de 1 ou 2 essais par semaines, les couples définitifs de souris qui seront utilisés pour les expériences. La sélection des couples est basée sur la stabilité de la

combativité des animaux d'un essai à l'autre, la faible latence de la première attaque et la fréquence et durée des attaques. Chez les couples ainsi sélectionnés, ces paramètres sont vérifiés chaque semaine au cours d'un essai rapide, sans traitement, deux jours avant le jour Test.

**Test :**

Le test a lieu une fois par semaine. Trente minutes avant leur mise en présence, les deux souris du couple reçoivent chacune le même traitement (produit ou solvant) et restent isolées dans leur cage respective. A T0., la souris intruse est introduite dans la cage de la souris résidente pour une durée de 3 minutes. On note la latence (en sec.) de la première attaque, le nombre et la durée totale (en sec.) des attaques. On note aussi une inversion éventuelle de la dominance d'une souris par rapport à l'autre (en général, la souris résidente est la souris dominante).
A la fin du test, la souris intruse retourne dans sa cage ; les animaux restent en isolement jusqu'au prochain essai rapide et test, la semaine suivante.

**Résultats :**

A titre d'exemple et pour illustrer l'activité des produits de l'invention, la dose inhibitrice 50, pour le composé de l'exemple 7 est inférieure à 1,5 mg/kg i.p.

***EXEMPLE 45 : Test d'enfouissement des billes chez la souris***

Ce test permet d'évaluer la capacité d'agents pharmacologiques à inhiber le comportement spontané d'enfouissement de billes chez la souris, cette inhibition étant prédictive d'action antidépressive et/ou anti-impulsive. Des souris mâles de souche NMRI (Iffa-Credo, l'Arbresle, France), pesant 20-25 g le jour de l'expérience, sont placées individuellement dans des boîtes en Macrolon (30 x 18 x 19 cm) contenant 5 cm de sciure et recouvertes par une plaque en plexiglass perforée. Vingt-quatre billes en verre "oeil-de-chat" sont réparties régulièrement sur la sciure à la périphérie de la boîte. Au terme de 30 minutes d'exploration libre, les animaux sont retirés de la boîte et le nombre de billes enfouies est comptabilisé.

**Résultats :**

A titre d'exemple, le tableau suivant montre les effets des produits de l'invention, en comparaison avec celui de la fluoxetine, un antidépresseur de référence.

| ***Exemple*** | ***Enfouissement des billes chez la souris $DI_{50}$*** |
|---|---|
| Fluoxétine | 8,03 |
| 7 | 1,64 |
| 11 | 0,94 |
| $DI_{50}$ = dose inhibitrice $_{50}$ | |

Les doses sont exprimées en mg/kg s.c.

***EXEMPLE 46 : Composition pharmaceutique : comprimés***

| Formule de préparation pour 1000 comprimés dosés à 5 mg | |
|---|---|
| Composé de l'exemple 7 | 5 g |
| Hydroxypropylméthylcellulose | 5 g |
| Amidon de blé | 10 g |
| Lactose | 100 g |
| Stéarate de Magnésium. | 2 g |

## Revendications

**1.** Composés de formule (I) :

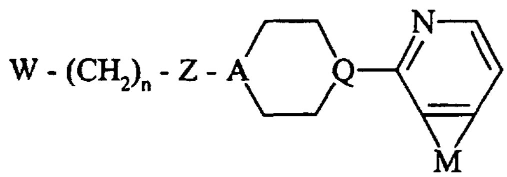

(I)

dans laquelle :

W représente :

- soit un groupement naphtyle éventuellement substitué, par un ou plusieurs groupements, identiques ou différents, choisis parmi halogène, alkyle ($C_1$-$C_6$) linéaire ou ramifié, hydroxy, alkoxy ($C_1$-$C_6$) linéaire ou ramifié, cyano, nitro, trihalogénoalkyle ($C_1$-$C_6$) linéaire ou ramifié, méthylènedioxy et éthylènedioxy,
- soit un groupement de formule Y telle que :

Y = 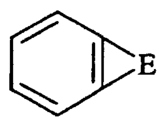

dans laquelle E représente, avec les atomes de carbone du cycle phényle auxquels il est lié, un système monocyclique, insaturé, partiellement saturé, ou aromatique, de 5 à 7 chaînons, comportant au moins un hétéroatome choisi parmi oxygène, azote et soufre, ledit groupement Y pouvant être lié au groupement $(CH_2)_n$ des composés de formule (I), soit par la partie phényle soit par le système monocyclique E, et chacun desdits groupements Y pouvant être éventuellement substitués par un ou plusieurs groupements, identiques ou différents, choisis parmi halogène, hydroxy, cyano, nitro, alkyle ($C_1$-$C_6$) linéaire ou ramifié, alkoxy ($C_1$-$C_6$) linéaire ou ramifié, trihalogénoalkyle ($C_1$-$C_6$) linéaire ou ramifié, hétérocycloalkylalkylène ($C_1$-$C_6$) linéaire ou ramifié (dans lequel l'hétérocycle est un système monocyclique, insaturé ou aromatique, de 5 à 6 chaînons, contenant de 1 à 4 hétéroatomes choisis parmi azote, soufre et oxygène), et oxo,
étant entendu que dans ce cas, le groupement Y ne peut être substitué que par un seul groupement oxo et que E représente alors, avec les atomes de carbone du cycle phényle auxquels il est lié, un monocycle à 5 chaînons contenant deux hétéroatomes, identiques ou différents, choisis parmi oxygène et azote,

n représente un entier compris entre 1 et 6 inclus,

Z représente une liaison simple, un atome d'oxygène ou un atome d'azote substitué par un groupement choisi parmi hydrogène, alkyle ($C_1$-$C_6$) linéaire ou ramifié (lui-même éventuellement substitué par un ou plusieurs groupements hydroxy), et arylalkyle ($C_1$-$C_6$) linéaire ou ramifié,

A représente un groupement CH ou un atome d'azote,

Q représente un groupement CH ou un atome d'azote,
étant entendu qu'au moins l'un des groupements A ou Q représente un atome d'azote, et que A représente un atome d'azote quand Z représente une liaison simple,

M représente, avec les atomes de carbone du cycle pyridinyle auxquels il est lié, un groupement thiéno, furo, pyrrolo ou oxopyrrolo,

ainsi que leurs sels d'addition à un acide ou à une base pharmaceutiquement acceptable.

**2.** Composés de formule (I) selon la revendication 1 **caractérisés en ce que** W représente un groupement choisi

parmi naphtyle, 2,3-dihydro-1,4-benzodioxinyle, chromanyle, 2*H*-chroményle, isochromanyle, 2,3-dihydrobenzofuranyle, benzofuranyle, indolyle, 1,3-dihydro-2*H*-benzimidazol-2-one, et 1,3-benzoxazol-2-one, ainsi que leurs sels d'addition à un acide ou à une base pharmaceutiquement acceptable.

**3.** Composés de formule (I) selon l'une quelconque des revendications 1 ou 2 **caractérisés en ce que** dans le cas où W est substitué par un groupement hétérocycloalkylalkylène ($C_1$-$C_6$) linéaire ou ramifié, alors ledit groupement hétérocycloalkylalkylène représente un groupement hétérocycloalkylméthylène dans lequel l'hétérocycle est un système monocyclique, insaturé ou aromatique, à 5 chaînons, contenant de 1 à 4 atomes d'azote, ainsi que leurs sels d'addition à un acide ou à une base pharmaceutiquement acceptable.

**4.** Composés de formule (I) selon la revendication 3 **caractérisés en ce que** W est substitué par un groupement hétérocycloalkylméthylène choisi parmi le groupement 1,2,4-triazol-1-ylméthyle, le groupement imidazol-1-ylméthyle, et le groupement 1*H*-imidazol-5-ylméthyle, ainsi que leurs sels d'addition à un acide ou à une base pharmaceutiquement acceptable.

**5.** Composés de formule (I) selon l'une quelconque des revendications 1 ou 2 **caractérisés en ce que** W représente un groupement choisi parmi 1-naphtyl, isochroman-1-yl et 2,3-dihydrobenzofuran-5-yl, ainsi que leurs sels d'addition à un acide ou à une base pharmaceutiquement acceptable.

**6.** Composés de formule (I) selon l'une quelconque des revendications 1 à 4 **caractérisés en ce que** W représente un groupement choisi parmi 5-(1,2,4-triazol-1-ylméthyl)-1*H*-indol-3-yl, 5-(imidazol-1-ylméthyl)-1*H*-indol-3-yl et 5-(1*H*-imidazol-5-ylméthyl)-1*H*-indol-3-yl, ainsi que leurs sels d'addition à un acide ou à une base pharmaceutiquement acceptable.

**7.** Composés de formule (I) selon l'une quelconque des revendications 1 ou 2 **caractérisés en ce que** W représente un groupement choisi parmi 1,3-benzoxazol-2-one-1-yl et 1,3-dihydro-2*H*-benzimidazole-2-one-1-yl, ainsi que leurs sels d'addition à un acide ou à une base pharmaceutiquement acceptable.

**8.** Composés de formule (I) selon la revendication 1 **caractérisés en ce que** Z représente une liaison simple, ainsi que leurs sels d'addition à un acide ou à une base pharmaceutiquement acceptable.

**9.** Composés de formule (I) selon la revendication 1 **caractérisés en ce que** Z représente un atome d'azote substitué par un groupement choisi parmi hydrogène ,méthyle ou hydroxyéthylène lorsque A représente un groupement CH, Q représente un atome d'azote, n prend la valeur I et W représente un groupement 2-naphtyle, ainsi que leurs sels d'addition à un acide ou à une base pharmaceutique acceptable.

**10.** Composés de formule (I) selon la revendication 1 **caractérisés en ce que** n représente 2 ou 3, ainsi que leurs sels d'addition à un acide ou à une base pharmaceutiquement acceptable.

**11.** Composés de formule (I) selon la revendication 1 **caractérisés en ce que** M représente, avec les atomes de carbone du cycle pyridinyle auxquels il est lié, un groupement thiényle ou furyle, ainsi que leurs sels d'addition à un acide ou à une base pharmaceutiquement acceptable.

**12.** Composé de formule (I) selon la revendication 1 qui est le 4-{1-[2-(1-naphtyl)éthyl]-4-pipéridinyl}thiéno[3,2-c]pyridine, ainsi que ses sels d'addition à un acide ou à une base pharmaceutiquement acceptable.

**13.** Composé de formule (I) selon la revendication 1 qui est le 4-{1-[2-(1-naphtyl)éthyl]-4-pipéridinyl}furo[3,2-c]pyridine, ainsi que ses sels d'addition à un acide ou à une base pharmaceutiquement acceptable.

**14.** Composé de formule (I) selon la revendication 1 qui est le 4-{4-[2-(2,3-dihydrobenzofuran-5-yl)éthyl]-1-pipérazinyl} furo[3,2-c]pyridine, ainsi que ses sels d'addition à un acide ou à une base pharmaceutiquement acceptable.

**15.** Composé de formule (I) selon la revendication 1 qui est le 4-{4-[2-(isochroman-1-yl)éthyl]-1-pipérazinyl}thiéno [3,2-c]pyridine, ainsi que ses sels d'addition à un acide ou à une base pharmaceutiquement acceptable.

**16.** Composés de formule (I) selon la revendication 1 qui sont le 4-(4-{2-[5-(1,2,4-triazol-1-ylméthyl)-1*H*-indol-3-yl] éthyl}-1-pipérazinyl)furo[3,2-c]pyridine, et le 4-(4-{2-[5-(1,2,4-triazol-1-ylméthyl)-1*H*-indol-3-yl]éthyl}-1-pipérazinyl)-1*H*-pyrrolo[3,2-c]pyridine, ainsi que leurs sels d'addition à un acide ou à une base pharmaceutiquement acceptable.

**17.** Composés de formule (I) selon la revendication 1 qui sont le 1-[2-(4-furo[3,2-c]pyridin-4-yl-1-pipérazinyl)éthyl]-1,3-dihydro-2*H*-benzimidazol-2-one et le 1-[2-(4-thiéno[3,2-c]pyridin-4-yl-1-pipérazinyl)éthyl]-1,3-dihydro-2*H*-benzimidazol-2-one, ainsi que leurs sels d'addition à un acide ou à une base pharmaceutiquement acceptable.

**18.** Procédé de préparation des composés de formule (I) **caractérisé en ce qu'**on utilise comme produit de départ, un composé de formule (II) :

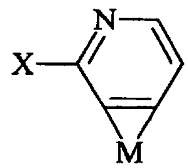

(II)

dans laquelle M est tel que défini dans la formule (I) et X représente un atome de chlore, de brome, d'iode, ou un groupe partant tel que mésyle, tosyle ou triflyle,

* composé de formule (II) que l'on fait réagir en présence d'une base avec un composé de formule (III) :

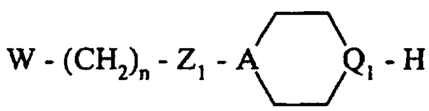

(III)

dans laquelle W, n et A sont tels que définis dans la formule (I), $Q_1$ représente un atome d'azote, et $Z_1$ représente une liaison simple, un atome d'oxygène ou un groupement NH,
pour conduire aux composés de formule (I/a), cas particulier des composés de formule (I) :

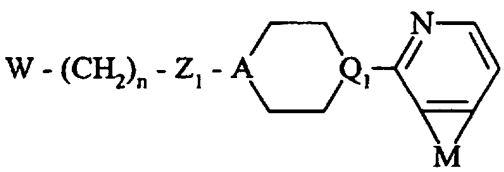

(I/a)

dans laquelle W, n, $Z_1$, A, M et $Q_1$ sont tels que définis précédemment,

* ou composés de formule (II) que l'on transforme, selon des conditions classiques de la synthèse organique, en composés de formule (IV) :

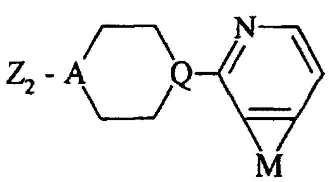

(IV)

dans laquelle A, Q et M sont tels que définis dans la formule (I), et $Z_2$ représente un atome d'hydrogène quand A représente un atome d'azote ou $Z_2$ représente un groupement $NH_2$ quand A représente un groupement CH, composés de formule (IV) que l'on fait réagir ,

- ***soit***, en présence d'un agent de couplage, avec un composé de formule (V) :

$$W - (CH_2)_{n-1} - CO_2H \qquad \textbf{(V)}$$

dans laquelle W et n sont tels que définis dans la formule (**I**), pour conduire aux composés de formule (VI) :

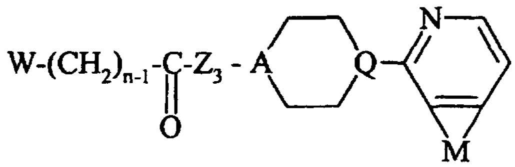

**(VI)**

dans laquelle W, n, A, Q et M sont tels que définis dans la formule (I), et $Z_3$ représente une liaison quand A représente un atome d'azote ou $Z_3$ représente un groupement NH quand A représente un groupement CH, composés de formule (VI) que l'on traite par un agent réducteur, selon des conditions classiques de la synthèse organique, pour conduire aux composés de formule (I/b), cas particulier des composés de formule (I) :

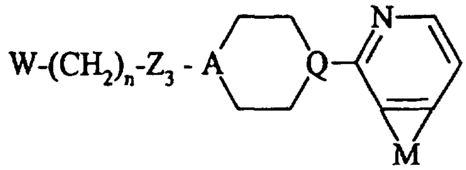

**(I/b)**

dans laquelle W, n, A, Q, M et $Z_3$ sont tels que définis précédemment,

- ***soit*** avec un composé de formule (VII), dans des conditions d'amination réductive :

$$W - (CH_2)_{n-1} - CHO \qquad \textbf{(VII)}$$

dans laquelle W et n sont tels que définis précédemment, pour conduire directement aux composés de formule (I/b) telle que définie précédemment,

- ***soit*** avec un composé de formule (VIII) en présence d'un agent de transfert de phase ou d'une base :

$$W - (CH_2)_n - X \qquad \textbf{(VIII)}$$

dans laquelle W et n sont tels que définis précédemment, et X représente un atome de chlore, de brome, d'iode, ou un groupement libérable tel que tosyle, mésyle ou triflyle, pour conduire également, directement, aux composés de formule (I/b) telle que définie précédemment, les composés de formules (I/a) et (I/b) formant les composés de formule (I/c) :

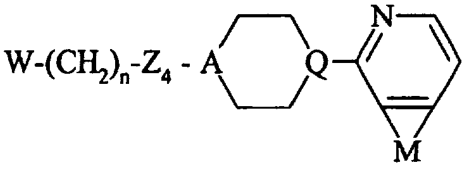

**(I/c)**

dans laquelle W, n, A, Q et M sont tels que définis dans la formule (I) et $Z_4$ représente une liaison, un atome d'oxygène ou un groupement NH, composés de formule (I/c), dans le cas particulier ou $Z_4$ prend la valeur $Z'_4$ et représente un groupement NH, et

A prend la valeur $A_1$ et représente un groupement CH, que l'on soumet à une réaction d'alkylation selon des méthodes classiques de la synthèse organique, pour conduire aux composés de formule (I/d), cas particulier des composés de formule (I) :

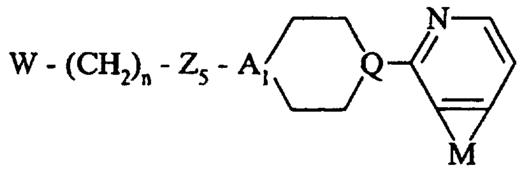

(I/d)

dans laquelle W, n, Q, M et $A_1$ sont tels que définis précédemment et $Z_5$ représente un atome d'azote substitué par un groupement alkyle ($C_1$-$C_6$) linéaire ou ramifié (lui-même éventuellement substitué par un ou plusieurs groupements hydroxy), ou arylalkyle ($C_1$-$C_6$) linéaire ou ramifé,
les composés (I/a) à (I/d) formant l'ensemble des composés de l'invention, que l'on purifie, le cas échéant, selon des techniques classiques de purification, dont on sépare éventuellement les isomères selon une technique classique de séparation, et que l'on transforme, si on le souhaite, en ses sels d'addition à un acide ou à une base pharmaceutiquement acceptable.

**19.** Compositions pharmaceutiques contenant comme principe actif au moins un composé de formule (I) selon l'une quelconque des revendications 1 à 17, seul ou en combinaison avec un ou plusieurs excipients ou véhicules inertes, non toxiques, pharmaceutiquement acceptables.

**20.** Compositions pharmaceutiques selon la revendication 19 contenant au moins un principe actif selon l'une quelconque des revendications 1 à 17, utiles pour le traitement de la dépression, de l'anxiété, des attaques de panique, des troubles obsessionnels compulsifs, des phobies, des troubles impulsifs et des troubles cognitifs.

## Patentansprüche

**1.** Verbindungen der Formel (I):

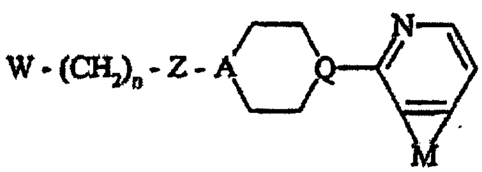

(I)

in der:

W:
- entweder eine Naphthylgruppe, die gegebenenfalls durch eine oder mehrere, gleichartige oder verschiedenartige Gruppen substituiert ist, ausgewählt aus Halogen, geradkettigem oder verzweigtem ($C_1$-$C_6$)-Alkyl, Hydroxy, geradkettigem oder verzweigtem ($C_1$-$C_6$)-Alkoxy, Cyano, Nitro, geradkettigem oder verzweigtem ($C_1$-$C_6$)-Trihalogenalkyl, Methylendioxy und Ethylendioxy,
- oder eine Gruppe der Formel Y bedeutet:

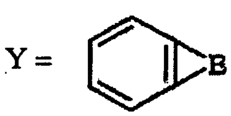

in der E zusammen mit den Kohlenstoffatomen des Phenylrings, an die sie gebunden ist, ein monocyclisches, ungesättigtes, teilweise gesättigtes oder aromatisches System mit 5 bis 7 Kettengliedern darstellt, welches mindestens ein Heteroatom ausgewählt aus Sauerstoff, Stickstoff und Schwefel enthält, wobei die Gruppe Y entweder über den Phenylrest oder über das monocyclische System E an die Gruppe $(CH_2)_n$ der Verbindungen der Formel (I) gebunden ist, und wobei jede der Gruppen Y gegebenenfalls durch eine oder

mehrere, gleichartige oder verschiedenartige Gruppen substituiert sein kann, ausgewählt aus Halogen, Hydroxy, Cyano, Nitro, geradkettigem oder verzweigtem ($C_1$-$C_6$)-Alkyl, geradkettigem oder verzweigtem ($C_1$-$C_6$)-Alkoxy, geradkettigem oder verzweigtem ($C_1$-$C_6$)-Trihalogenalkyl, geradkettigem oder verzweigtem Heterocycloalkyl-($C_1$-$C_6$)-alkylen (worin der Heterocyclus ein monocyclisches, ungesättigtes oder aromatisches System mit 5 bis 6 Kettengliedern darstellt, welches 1 bis 4 Heteroatome, ausgewählt aus Stickstoff, Schwefel und Sauerstoff enthält) und Oxo,
mit der Maßgabe, daß in diesem Fall die Gruppe Y nur durch eine einzige Oxogruppe substituiert sein kann und E dann mit den Kohlenstoffatomen des Phenylrings, an die sie gebunden ist, einen Monocyclus mit 5 Kettengliedern, der zwei gleichartige oder verschiedene Heteroatome ausgewählt aus Sauerstoff und Stickstoff enthält, bedeutet,

n eine ganze Zahl von 1 bis 6 einschließlich darstellt,
Z eine Einfachbindung, ein Sauerstoffatom oder ein Stickstoffatom, welches durch eine Gruppe substituiert ist, ausgewählt aus Wasserstoff, geradkettigem oder verzweigtem ($C_1$-$C_6$)-Alkyl (welches seinerseits durch eine oder mehrere Hydroxygruppen substituiert ist) und geradkettigem oder verzweigtem Aryl-($C_1$-$C_6$)-alkyl, bedeutet,
A eine Gruppe CH oder eine Stickstoffatom bedeutet,
Q eine Gruppe CH oder ein Stickstoffatom bedeutet, mit der Maßgabe, daß mindestens eine der Gruppen A oder Q eine Stickstoffatom darstellt und A ein Stickstoffatom bedeutet, wenn Z eine Einfachbindung darstellt, und
M zusammen mit den Kohlenstoffatomen des Pyridinylrings, an die es gebunden ist, eine Thieno-, Furo-, Pyrrolo- oder Oxopyrrolo-gruppe bedeutet,

und deren Additionssalze mit einer pharmazeutisch annehmbaren Säure oder Base.

**2.** Verbindungen der Formel (I) nach Anspruch 1, **dadurch gekennzeichnet, daß** W eine Gruppe bedeutet ausgewählt aus Naphthyl, 2,3-Dihydro-1,4-benzodioxinyl, Chromanyl, 2*H*-Chromenyl, Isochromanyl, 2,3-Dihydrobenzofuranyl, Benzofuranyl, Indolyl, 1,3-Dihydro-2*H*-benzimidazol-2-on und 1,3-Benzoxazol-2-on, sowie deren Additionssalze mit einer pharmazeutisch annehmbaren Säure oder Base.

**3.** Verbindungen der Formel (I) nach einem der Ansprüche 1 oder 2, **dadurch gekennzeichnet, daß** dann, wenn W durch eine geradkettige oder verzweigte Heterocycloalkyl-($C_1$-$C_6$)-alkylengruppe substituiert ist, die Heterocycloalkylalkylengruppe eine Heterocycloalkylmethylengruppe darstellt, bei der der Heterocyclus ein ungesättigtes oder aromatisches monocyclisches System mit 5 Kettengliedern darstellt, welches 1 bis 4 Stickstoffatome enthält, sowie deren Additionssalze mit einer pharmazeutisch annehmbaren Säure oder Base.

**4.** Verbindungen der Formel (I) nach Anspruch 3, **dadurch gekennzeichnet, daß** W durch eine Heterocycloalkylmethylengruppe substituiert ist, ausgewählt aus 1,2,4-Triazol-1-ylmethyl-gruppe, der Imidazol-1-ylmethyl-gruppe und der 1*H*-Imidazol-5-ylmethyl-gruppe, sowie deren Additionssalze mit einer pharmazeutisch annehmbaren Säure oder Base.

**5.** Verbindungen der Formel (I) nach einem der Ansprüche 1 oder 2, **dadurch gekennzeichnet, daß** W eine Gruppe bedeutet ausgewählt aus 1-Naphthyl, Isochroman-1-yl und 2,3-Dihydrobenzofuran-5-yl, sowie deren Additionssalze mit einer pharmazeutisch annehmbaren Säure oder Base.

**6.** Verbindungen der Formel (I) nach einem der Ansprüche 1 bis 4, **dadurch gekennzeichnet, daß** W eine Gruppe bedeutet ausgewählt aus 5-(1,2,4-Triazol-1-ylmethyl)-1*H*-indol-3-yl, 5-(Imidazol-1-ylmethyl)-1*H*-indol-3-yl und 5-(1*H*-Imidazol-5-ylmethyl)-1*H*-indol-3-yl, sowie deren Additionssalze mit einer pharmazeutisch annehmbaren Säure oder Base.

**7.** Verbindungen der Formel (I) nach einem der Ansprüche 1 oder 2, **dadurch gekennzeichnet, daß** W eine Gruppe bedeutet ausgewählt aus 1,3-Benzoxazol-2-on-1-yl und 1,3-Dihydro-2*H*-benzimidazol-2-on-1-yl, sowie deren Additionssalze mit einer pharmazeutisch annehmbaren Säure oder Base.

**8.** Verbindungen der Formel (I) nach Anspruch 1, **dadurch gekennzeichnet, daß** Z eine Einfachbindung bedeutet, sowie deren Additionssalze mit einer pharmazeutisch annehmbaren Säure oder Base.

**9.** Verbindungen der Formel (I) nach Anspruch 1, **dadurch gekennzeichnet, daß** Z ein Stickstoffatom bedeutet,

welches durch eine Gruppe ausgewählt aus Wasserstoff, Methyl oder Hydroxyethylen substituiert ist, wenn A eine Gruppe CH, Q ein Stickstoffatom, n den Wert 1 und W eine 2-Naphthylgruppe bedeuten, sowie deren Additionssalze mit einer pharmazeutisch annehmbaren Säure oder Base.

**10.** Verbindungen der Formel (I) nach Anspruch 1, **dadurch gekennzeichnet, daß** n den Wert 2 oder 3 besitzt, sowie deren Additionssalze mit einer pharmazeutisch annehmbaren Säure oder Base.

**11.** Verbindungen der Formel (I) nach Anspruch 1, **dadurch gekennzeichnet, daß** M zusammen mit den Kohlenstoffatomen des Pyridinylrings, an die es gebunden ist, eine Thienyl- oder Furyl-gruppe bedeutet, sowie deren Additionssalze mit einer pharmazeutisch annehmbaren Säure oder Base.

**12.** Verbindung der Formel (I) nach Anspruch 1, nämlich 4-{1-[2-(1-Naphthyl)-ethyl]-4-piperidinyl}-thieno[3,2-c]pyridin, sowie dessen Additionssalze mit einer pharmazeutisch annehmbaren Säure oder Base.

**13.** Verbindung der Formel (I) nach Anspruch 1, nämlich 4-{1-[2-(1-Naphthyl)-ethyl]-4-piperidinyl}-furo[3,2-c]pyridin, sowie dessen Additionssalze mit einer pharmazeutisch annehmbaren Säure oder Base.

**14.** Verbindung der Formel (I) nach Anspruch 1, nämlich 4-{4-[2-(2,3-Dihydrobenzofuran-5-yl)-ethyl]-1-piperazinyl}-furo[3,2-c]pyridin, sowie dessen Additionssalze mit einer pharmazeutisch annehmbaren Säure oder Base.

**15.** Verbindung der Formel (I) nach Anspruch 1, nämlich 4-{4-[2-(Isochroman-1-yl)-ethyl]-1-piperazinyl}-thieno[3,2-c] pyridin, sowie dessen Additionssalze mit einer pharmazeutisch annehmbaren Säure oder Base.

**16.** Verbindungen der Formel (I) nach Anspruch 1, nämlich 4-(4-{2-[5-(1,2,4-Triazol-1-ylmethyl)-1*H*-indol-3-yl]-ethyl}-1-piperazinyl)-furo[3,2-c]pyridin und 4-(4-{2-[5-(1,2,4-Triazol-1-ylmethyl)-1*H*-indol-3-yl]-ethyl}-1-piperazinyl)-1*H*-pyrrolo[3,2-c]pyridin, sowie deren Additionssalze mit einer pharmazeutisch annehmbaren Säure oder Base.

**17.** Verbindungen der Formel (I) nach Anspruch 1, nämlich 1-[2-(4-Furo[3,2-c]pyridin-4-yl-1-piperazinyl)-ethyl]-1,3-dihydro-2*H*-benzimidazol-2-on und 1-[2-(4-Thieno[3,2-c]pyridin-4-yl-1-piperazinyl)-ethyl]-1,3-dihydro-2*H*-benzimidazol-2-on, sowie deren Additionssalze mit einer pharmazeutisch annehmbaren Säure oder Base.

**18.** Verfahren zur Herstellung der Verbindungen der Formel (I), **dadurch gekennzeichnet, daß** man als Ausgangsprodukt eine Verbindung der Formel (II) verwendet:

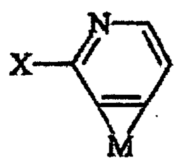

(II)

in der M die bezüglich der Formel (I) angegebenen Bedeutungen besitzt und X ein Chlor-, Brom oder Iodatom oder eine austretende Gruppe, wie eine Mesyl-, Tosyl- oder Triflyl-gruppe bedeutet,

- welche Verbindung der Formel (II) man in Gegenwart einer Base mit einer Verbindung der Formel (III) umsetzt:

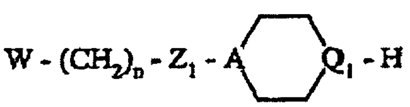

(III)

in der W, n und A die bezüglich der Formel (I) angegebenen Bedeutungen besitzen, $Q_1$ ein Stickstoffatom und $Z_1$ eine Einfachbindung, ein Sauerstoffatom oder eine Gruppe NH bedeuten,
zur Bildung der Verbindungen der Formel (I/a), einem Sonderfall der Verbindungen der Formel (I):

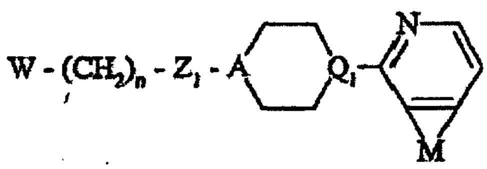

(I/a)

in der W, n, $Z_1$, A, M und $Q_1$ die oben angegebenen Bedeutungen besitzen,

* oder man die Verbindungen der Formel (II) unter klassischen Bedingungen der organischen Synthese in die Verbindungen der Formel (IV) umwandelt:

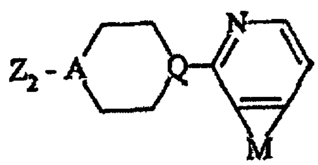

(IV)

in der A, Q und M die bezüglich der Formel (I) angegebenen Bedeutungen besitzen und $Z_2$ ein Wasserstoffatom bedeutet, wenn A ein Stickstoffatom darstellt, oder $Z_2$ eine Gruppe $NH_2$ darstellt, wenn A eine Gruppe CH bedeutet, welche Verbindungen der Formel (IV) man

- ***entweder*** in Gegenwart eines Kupplungsmittels mit einer Verbindung der Formel (V) umsetzt:

$$W - (CH_2)_{n-1} - CO_2H \qquad \textbf{(V)}$$

in der W und n die bezüglich der Formel (I) angegebenen Bedeutungen besitzen, zur Bildung der Verbindungen der Formel (VI):

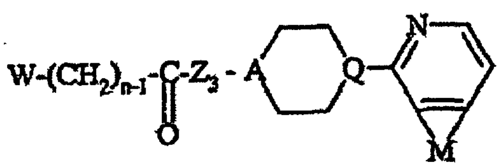

(VI)

in der W, n, A, Q und M die bezüglich der Formel (I) angegebenen Bedeutungen besitzen und $Z_3$ eine Bindung darstellt, wenn A ein Stickstoffatom bedeutet, oder $Z_3$ eine Gruppe NH darstellt, wenn A eine Gruppe CH bedeutet, welche Verbindungen der Formel (VI) mit einem Reduktionsmittel unter klassischen Bedingungen der organischen Synthese behandelt zur Bildung der Verbindungen der Formel (I/b), einem Sonderfall der Verbindungen der Formel (I):

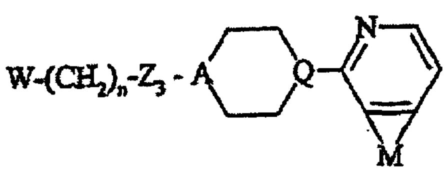

(I/b)

in der W, n, A, Q, M und $Z_3$ die oben angegebenen Bedeutungen besitzen,

- ***oder*** mit einer Verbindung der Formel (VII) unter den Bedingungen der reduktiven Aminierung umsetzt;

$$W - (CH_2)_{n-1} - CHO \qquad \textbf{(VII)}$$

in der W und n die oben angegebenen Bedeutungen besitzen, zur direkten Bildung der Verbindungen der Formel (I/b), wie sie oben definiert worden ist,

- ***oder*** mit einer Verbindung der Formel (VIII) in Gegenwart eines Phasentransfermittels oder einer Base umsetzt:

$$W - (CH_2)_n - X \qquad \textbf{(VIII)}$$

in der W und n die oben angegebenen Bedeutungen besitzen und X ein Chlor-, Brom- oder Iodatom oder eine freisetzbare Gruppe, wie eine Tosyl-, Mesyl- oder Triflyl-gruppe bedeutet, zur ebenfalls direkten Bildung der Verbindungen der Formel (I/b), wie sie oben definiert worden ist, wobei die Verbindungen der Formeln (I/a) und (I/b) die Verbindungen der Formel (I/c) bilden:

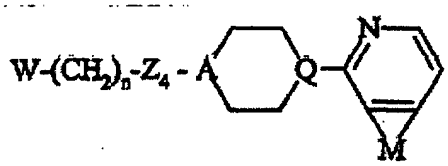

**(I/c)**

in der W, n, A, Q und M die bezüglich der Formel (I) angegebenen Bedeutungen besitzen und $Z_4$ eine Bindung, ein Sauerstoffatom oder eine NH-Gruppe bedeutet, welche Verbindungen der Formel (I/c) man, wenn $Z_4$ die Bedeutung $Z'_4$ besitzt und eine NH-Gruppe bedeutet und A den Wert $A_1$ besitzt und eine Gruppe CH bedeutet, einer Alkylierungsreaktion gemäß klassischen Methoden der organischen Synthese unterwirft zur Bildung der Verbindungen der Formel (I/d), einem Sonderfall der Verbindungen der Formel (I):

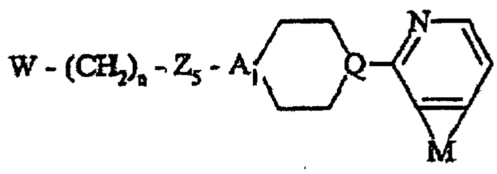

**(I/d)**

in der W, n, Q, M und $A_1$ die oben angegebenen Bedeutungen besitzen und $Z_5$ ein Stickstoffatom bedeutet, welches durch eine geradkettige oder verzweigte ($C_1$-$C_6$)-Alkylgruppe (die ihrerseits durch eine oder mehrere Hydroxygruppen substituiert sein kann) oder eine geradkettige oder verzweigte Aryl-($C_1$-$C_6$)-alkylgruppe substituiert ist, wobei man die Verbindungen (I/a) bis (I/d), welche die Gesamtheit der erfindungsgemäßen Verbindungen bilden, gegebenenfalls mit Hilfe klassischer Reinigungsmethoden reinigt, gegebenenfalls mit Hilfe einer klassischen Trennungsmethode in ihre eventuellen Isomeren auftrennt und gewünschtenfalls in ihre Additionssalze mit einer pharmazeutisch annehmbaren Säure oder Base umwandelt.

**19.** Pharmazeutische Zubereitungen, enthaltend als Wirkstoff mindestens eine Verbindung der Formel (I) nach einem der Ansprüche 1 bis 17 allein oder in Kombination mit einem oder mehreren inerten, nichttoxischen, pharmazeutisch annehmbaren Bindemitteln oder Trägermaterialien.

**20.** Pharmazeutische Zubereitungen nach Anspruch 19, enthaltend mindestens einen Wirkstoff nach einem der Ansprüche 1 bis 17 zur Behandlung der Depression, der Angst, von Panikanfällen, von krampfartigen Besessenheitsstörungen, von Phobien, impulsiven Störungen und Erkenntnisstörungen.

## Claims

**1.** Compounds of formula (I) :

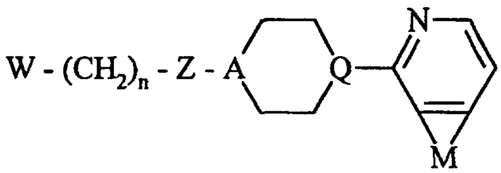

(I)

wherein:

W represents :
- either a naphthyl group optionally substituted by one or more identical or different groups selected from halogen, linear or branched ($C_1$-$C_6$)alkyl, hydroxy, linear or branched ($C_1$-$C_6$) alkoxy, cyano, nitro, linear or branched trihalo($C_1$-$C_6$)alkyl, methylenedioxy and ethylenedioxy,
- or a group of formula Y where :

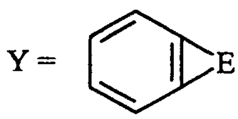

wherein E, together with the carbon atoms of the phenyl ring to which it is bonded, represents an unsaturated, partially saturated, or aromatic, monocyclic ring having from 5 to 7 ring members and containing at least one hetero atom selected from oxygen, nitrogen and sulphur, it being possible for the said group Y to be bonded to the $(CH_2)_n$ group of the compounds of formula (I) either by the phenyl moiety or by the monocyclic ring E, and for each of the said Y groups to be optionally substituted by one or more identical or different groups selected from halogen, hydroxy, cyano, nitro, linear or branched ($C_1$-$C_6$)alkyl, linear or branched ($C_1$-$C_6$)alkoxy, linear or branched trihalo($C_1$-$C_6$)alkyl, heterocycloalkylalkylene in which the alkylene moiety contains from 1 to 6 carbon atoms and may be linear or branched (and in which the heterocyclic ring is an unsaturated or aromatic monocyclic ring having 5 or 6 ring members and containing from 1 to 4 hetero atoms selected from nitrogen, sulphur and oxygen) and oxo,
provided that in that case the group Y can be substituted only by a single oxo group and that E, in that case, together with the carbon atoms of the phenyl ring to which it is bonded, represents a monocyclic ring having 5 ring members and containing two identical or different hetero atoms selected from oxygen and nitrogen,

n represents an integer from 1 to 6 inclusive,

Z represents a single bond, an oxygen atom, or a nitrogen atom substituted by a group selected from hydrogen, linear or branched ($C_1$-$C_6$)alkyl (itself optionally substituted by one or more hydroxy groups) and aryl-($C_1$-$C_6$)alkyl in which the alkyl moiety may be linear or branched,

A represents a CH group or a nitrogen atom,

Q represents a CH group or a nitrogen atom,
it being understood that at least one of the groups A and Q represents a nitrogen atom, and that A represents a nitrogen atom when Z represents a single bond, and

M, together with the carbon atoms of the pyridinyl ring to which it is bonded, represents a thieno, furo, pyrrolo or oxopyrrolo group,

and also addition salts thereof with a pharmaceutically acceptable acid or base.

**2.** Compounds of formula (I) according to claim 1, **characterised in that** W represents a group selected from naphthyl, 2,3-dihydro-1,4-benzodioxinyl, chromanyl, 2*H*-chromenyl, isochromanyl, 2,3-dihydrobenzofuranyl, benzofuranyl, indolyl, 1,3-dihydro-2*H*-benzimidazol-2-one and 1,3-benzoxazol-2-one, and also addition salts thereof with a pharmaceutically acceptable acid or base.

**3.** Compounds of formula (I) according to either claim 1 or claim 2, **characterised in that** when W is substituted by a heterocycloalkylalkylene group in which the alkylene moiety contains from 1 to 6 carbon atoms and may be linear or branched, the said heterocycloalkylalkylene group is a heterocycloalkylmethylene group in which the heterocyclic ring is an unsaturated or aromatic monocyclic ring having 5 ring members and containing from 1 to 4 nitrogen atoms, and also addition salts thereof with a pharmaceutically acceptable acid or base.

**4.** Compounds of formula (I) according to claim 3, **characterised in that** W is substituted by a heterocycloalkylmethylene group selected from the 1,2,4-triazol-1-ylmethyl group, the imidazol-1-ylmethyl group and the 1*H*-imidazol-5-ylmethyl group, and also addition salts thereof with a pharmaceutically acceptable acid or base.

**5.** Compounds of formula (I) according to either claim 1 or claim 2, **characterised in that** W represents a group selected from 1-naphthyl, isochroman-1-yl and 2,3-dihydrobenzofuran-5-yl, and also addition salts thereof with a pharmaceutically acceptable acid or base.

**6.** Compounds of formula (I) according to any one of claims 1 to 4, **characterised in that** W represents a group selected from 5-(1,2,4-triazol-1-ylmethyl)-1*H*-indol-3-yl, 5-(imidazol-1-ylmethyl)-1*H*-indol-3-yl and 5-(1*H*-imidazol-5-ylmethyl)-1*H*-indol-3-yl, and also addition salts thereof with a pharmaceutically acceptable acid or base.

**7.** Compounds of formula (I) according to either claim 1 or claim 2, **characterised in that** W represents a group selected from 1,3-benzoxazol-2-on-1-yl and 1,3-dihydro-2*H*-benzimidazol-2-on-1-yl, and also addition salts thereof with a pharmaceutically acceptable acid or base.

**8.** Compounds of formula (I) according to claim 1, **characterised in that** Z represents a single bond, and also addition salts thereof with a pharmaceutically acceptable acid or base.

**9.** Compounds of formula (I) according to claim 1, **characterised in that**, when A represents a CH group, Q represents a nitrogen atom, n is 1 and W represents a 2-naphthyl group, Z represents a nitrogen atom substituted by a group selected from hydrogen, methyl and hydroxyethylene, and also addition salts thereof with a pharmaceutically acceptable acid or base.

**10.** Compounds of formula (I) according to claim 1, **characterised in that** n represents 2 or 3, and also addition salts thereof with a pharmaceutically acceptable acid or base.

**11.** Compounds of formula (I) according to claim 1, **characterised in that** M, together with the carbon atoms of the pyridinyl ring to which it is bonded, represents a thienyl or furyl group, and also addition salts thereof with a pharmaceutically acceptable acid or base.

**12.** Compound of formula (I) according to claim 1 that is 4-{1-[2-(1-naphthyl)ethyl]-4-piperidinyl}thieno[3,2-c]pyridine, and also addition salts thereof with a pharmaceutically acceptable acid or base.

**13.** Compound of formula (I) according to claim 1 that is 4-{1-[2-(1-naphthyl)ethyl]-4-piperidinyl}furo[3,2-c]pyridine, and also addition salts thereof with a pharmaceutically acceptable acid or base.

**14.** Compound of formula (I) according to claim 1 that is 4-{4-[2-(2,3-dihydrobenzofuran-5-yl)ethyl]-1-piperazinyl}furo[3,2-c]pyridine, and also addition salts thereof with a pharmaceutically acceptable acid or base.

**15.** Compound of formula (I) according to claim 1 that is 4-{4-[2-(isochroman-1-yl)ethyl]-1-piperazinyl}thieno[3,2-c]pyridine, and also addition salts thereof with a pharmaceutically acceptable acid or base.

**16.** Compounds of formula (I) according to claim 1 that are 4-(4-{2-[5-(1,2,4-triazol-1-ylmethyl)-1*H*-indol-3-yl]ethyl}-1-piperazinyl)furo[3,2-c]pyridine and 4-(4-{2-[5-(1,2,4-triazol-1-ylmethyl)-1*H*-indol-3-yl]ethyl}-1-piperazinyl)-1*H*-pyrrolo[3,2-c]pyridine, and also addition salts thereof with a pharmaceutically acceptable acid or base.

**17.** Compounds of formula (I) according to claim 1 that are 1-[2-(4-furo[3,2-c]pyridin-4-yl-1-piperazinyl)ethyl]-1,3-dihydro-2*H*-benzimidazol-2-one and 1-[2-(4-thieno[3,2-c]-pyridin-4-yl-1-piperazinyl)ethyl]-1,3-dihydro-2*H*-benzimidazol-2-one, and also addition salts thereof with a pharmaceutically acceptable acid or base.

**18.** Process for the preparation of the compounds of formula (I), **characterised in that** there is used as starting material a compound of formula (II) :

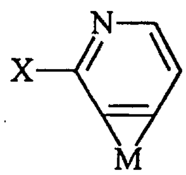

(II)

wherein M is as defined for formula (I) and X represents a chlorine, bromine or iodine atom or a leaving group, such as mesyl, tosyl or triflyl,

* which compound of formula (II) is reacted in the presence of a base with a compound of formula (III) :

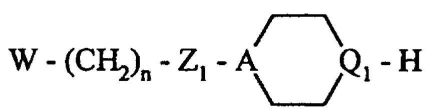

(III)

wherein W, n and A are as defined for formula (I), $Q_1$ represents a nitrogen atom and $Z_1$ represents a single bond, an oxygen atom or an NH group,
to yield the compounds of formula (I/a), a particular case of the compounds of formula (I) :

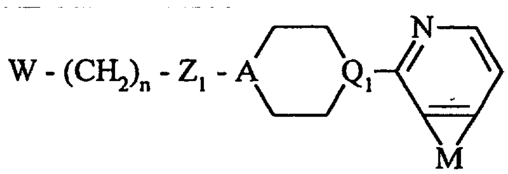

(I/a)

wherein W, n, $Z_1$, A, M and $Q_1$ are as defined hereinbefore,

* or which compounds of formula (II) are converted, in accordance with conventional conditions of organic synthesis, into compounds of formula (IV) :

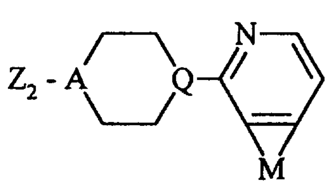

(IV)

wherein A, Q and M are as defined for formula (I), and $Z_2$ represents a hydrogen atom when A represents a nitrogen atom or $Z_2$ represents an $NH_2$ group when A represents a CH group,
which compounds of formula (IV) are reacted,

- ***either*** with a compound of formula (V) :

$$W - (CH_2)_{n-1} - CO_2H \qquad \textbf{(V)}$$

wherein W and n are as defined for formula (I), in the presence of a coupling agent, to yield the compounds of formula (VI) :

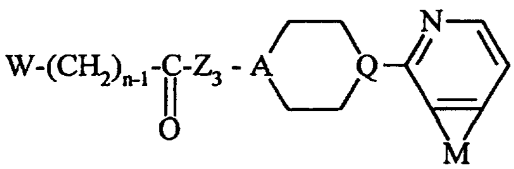

**(VI)**

wherein W, n, A, Q and M are as defined for formula (I), and $Z_3$ represents a bond when A represents a nitrogen atom or $Z_3$ represents an NH group when A represents a CH group,
which compounds of formula (VI) are treated with a reducing agent, in accordance with conventional conditions of organic synthesis, to yield the compounds of formula (I/b), a particular case of the compounds of formula (I) :

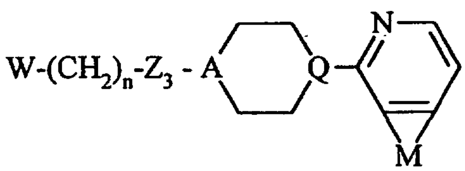

**(I/b)**

wherein W, n, A, Q, M and $Z_3$ are as defined hereinbefore,

- ***or*** with a compound of formula (VII):

$$W - (CH_2)_{n-1} - CHO \qquad \textbf{(VII)}$$

wherein W and n are as defined hereinbefore, under reductive amination conditions, to yield, directly, the compounds of formula (I/b) as defined hereinbefore,

- ***or*** with a compound of formula (VIII):

$$W - (CH_2)_n - X \qquad \textbf{(VIII)}$$

wherein W and n are as defined hereinbefore and X represents a chlorine, bromine or iodine atom or a leaving group, such as tosyl, mesyl or triflyl, in the presence of a phase transfer agent or a base,
also to yield, directly, the compounds of formula (I/b) as defined hereinbefore,
the compounds of formulae (I/a) and (I/b) constituting the compounds of formula (I/c) :

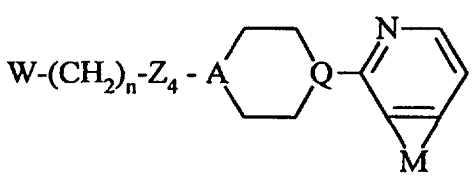

**(I/c)**

wherein W, n, A, Q and M are as defined for formula (I) and $Z_4$ represents a bond, an oxygen atom or an NH group, which compounds of formula (I/c), when $Z_4$ is $Z'_4$ and represents an NH group, and A is $A_1$ and represents a CH group, are subjected to an alkylation reaction according to conventional methods of organic synthesis to yield the

compounds of formula (I/d), a particular case of the compounds of formula (I) :

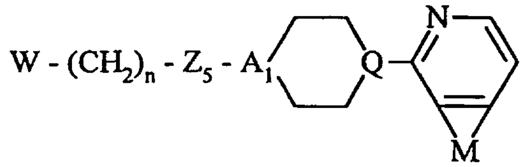

(I/d)

wherein W, n, Q, M and $A_1$ are as defined hereinbefore and $Z_5$ represents a nitrogen atom substituted by a linear or branched ($C_1$-$C_6$)alkyl group (itself optionally substituted by one or more hydroxy groups) or by aryl-($C_1$-$C_6$) alkyl in which the alkyl moiety may be linear or branched,
the compounds (I/a) to (I/d) constituting the totality of the compounds of the invention, which compounds are purified, if necessary, according to conventional purification techniques, are separated, if desired, into the isomers according to a conventional separation technique, and are converted, if desired, into addition salts with a pharmaceutically acceptable acid or base.

**19.** Pharmaceutical compositions comprising as active ingredient at least one compound of formula (I) according to any one of claims 1 to 17, alone or in combination with one or more pharmaceutically acceptable, inert, non-toxic excipients or carriers.

**20.** Pharmaceutical compositions according to claim 19 comprising at least one active ingredient according to any one of claims 1 to 17 for use in the treatment of depression, anxiety, panic attacks, obsessive-compulsive disorders, phobias, impulsive disorders and cognitive disorders.